# EUROPEAN PATENT APPLICATION

(11) **EP 4 243 029 A1**
(43) Date of publication of application: **13.09.2023**
(21) Application number: 22163169.0
(22) Date of filing: 21.03.2022
(51) Int. Cl.: G16H 15/00, G16H 30/40, G16H 40/20

(54) **MEDICAL IMAGING PROCESSING SYSTEM**

(30) Priority: 08.03.2022 US 202263317665 P
(71) Applicant: Koninklijke Philips N.V., 5656 AG Eindhoven (NL)
(72) Inventor: BUIL, Vincentius Paulus, Eindhoven (NL); VAN OORDE-GRAINGER, Shaun, Eindhoven (NL); HINGSTON, John, Eindhoven (NL); KNOESTER, Jaap, Eindhoven (NL)
(74) Representative: Philips Intellectual Property & Standards

(57) **Abstract**

The present invention relates to a medical imaging processing system (10), comprising: a first virtual assistant (20); a second virtual assistant (30, 40); and a third virtual assistant (50). The first virtual assistant is configured to support a physician to prepare an imaging request for a patient, wherein the imaging request is to be used by a technician who will carry out an imaging exam of the patient to generate patient imagery, and wherein the first virtual assistant is configured to utilize one or more of the following to support the physician to prepare the imaging request: information on the patient, information from a medical database, information on the physician, information on previous imaging requests, the physician's response to dialogue with the first virtual assistant, wherein the dialogue with the first virtual assistant comprises utilization of a VDU and/or a chatbot implemented by the first virtual assistant. The second virtual assistant is configured to support the technician to carry out the imaging exam of the patient according to the imaging request to generate the patient imagery, and wherein the second virtual assistant is configured to utilize one or more of the following to support the technician to carry out the imaging exam of the patient according to the imaging request: information on the patient, information on the technician, information on a medical expert who will review the patient imagery and generate a report for the physician. The third virtual assistant is configured to support the medical expert to review the patient imagery and generate the report for the physician, and wherein the third virtual assistant is configured to utilize one or more of the following to support the medical expert to review the patient imagery and generate the report for the physician: information on the patient, information on the medical expert, information on previous reports generated from patient imagery, the medical expert's response to dialogue with the third virtual assistant, wherein the dialogue with the third virtual assistant comprises utilization of a VDU and/or a chatbot implemented by the third virtual assistant.

## Description

### FIELD OF THE INVENTION

The present invention relates to medical imaging processing systems, medical imaging processing methods, as well as to a computer program element and a computer readable medium.

### BACKGROUND OF THE INVENTION

To set the background to the invention, below is laid out details of the medical imaging process.

### Radiology:

Radiology is the medical discipline that uses medical imaging to diagnose and treat diseases within the bodies of animals and humans. A variety of imaging techniques such as X-ray radiography, ultrasound, computed tomography (CT), nuclear medicine including positron emission tomography (PET), fluoroscopy, and magnetic resonance imaging (MRI) are used to diagnose or treat diseases. The modern practice of radiology involves several different healthcare professions working as a team.
More detail can be found at: https://en.wikipedia.org/wiki/Radiology.

Thus, radiology technicians can set up and take all manner of imaging exams and a radiologist can assess that imaging. Such a generic radiology technician can also be termed a technician and such a generic radiologist can be termed a medical expert. However, there are also technicians who specialize in X-ray, MRI, PET or ultrasound and similarly medical experts who specialize in X-ray, MRI, PET or ultrasound.

### Referring Physician

The Referring Physician is normally the patient facing physician, who after consultation with the patient, requests an imaging procedure. Common referring Physicians include:
General Practitioners (GP)
Medical Specialists
Emergency Room (ER) physicians

### Diagnostic Radiologists:

'Diagnostic Radiologists', or sometimes called 'Radiologist' for short, are medical doctors that specialize in diagnosing injuries and diseases using medical imaging (radiology) procedures (exams/tests) such as X-rays, computed tomography (CT), magnetic resonance imaging (MRI), nuclear medicine, positron emission tomography (PET) and ultrasound. The radiologist is the doctor who supervises these exams, reads and interprets the images, and writes a report for the referring physician. As detailed above, this person is here also termed a medical expert.

More detail can be found at: https://www.acr.org/Practice-Management-Quality-Informatics/Practice-Toolkit/Patient-Resources/About-Radiology

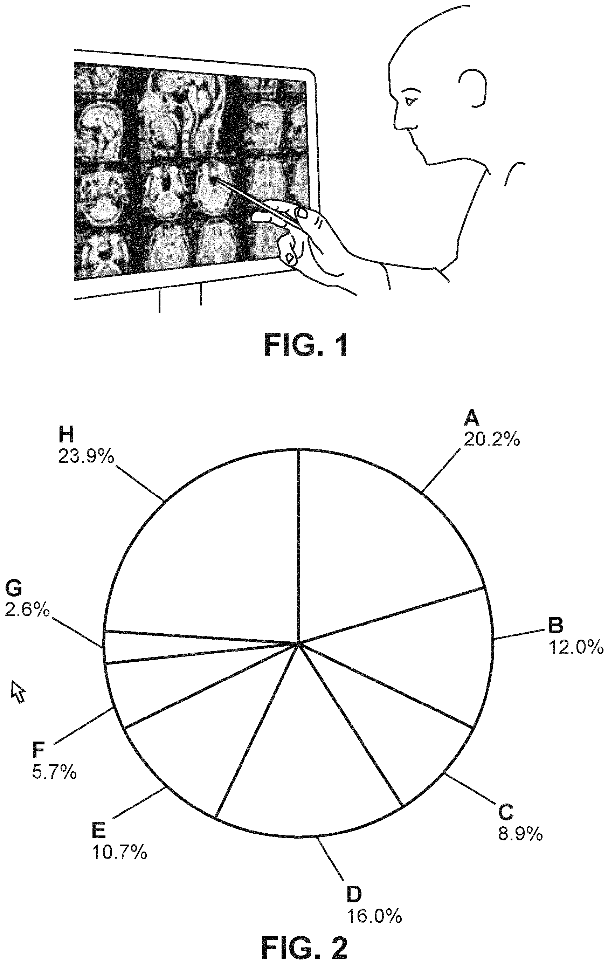

Fig. 1 shows an example of a diagnostic radiologist "reading" medical imagery or images to diagnose and document what is seen. The diagnostic radiologist could for example be using a dictation system to dictate a report that would then be sent back to the referring physician.

Diagnostic radiologists use a variety of imaging procedures to see inside the body to assess or diagnose the patient's condition. Radiologists play an important role as the expert consultant to the referring physician by providing assistance in choosing the proper exam and directing radiology technologists (those who operate the equipment) in properly performing quality exams. Diagnostic radiologists interpret and report on the resulting images, recommending treatment and, only when appropriate, additional tests. Diagnostic radiologists, may specialize in the radiology subspecialties. Below are examples of specialists:
Breast imaging (mammograms)
Cardiovascular radiology (heart and circulatory system)
Chest radiology (heart and lungs)
Emergency radiology
Gastrointestinal radiology (stomach, intestines and abdomen)
Genitourinary radiology (reproductive and urinary systems)
Head and neck radiology
Musculoskeletal radiology (muscles and skeleton)
Neuroradiology (brain and nervous system; head, neck and spine)
Pediatric radiology (imaging of children)

Fig. 2 presents an indication of the medical imaging Informatics market, with respect to revenue percentage forecast by segments. This has been sourced from "Global Medical Imaging & Informatics Outlook, 2021, Frost & Sullivan". In Fig. 2 "ultrasound" is represented by A, "MR" is represented by B, "x-ray radiography (only CR and DR)" is represented by C, "CT" is represented by D, "interventional x-ray (IXR, C-arm)" is represented by E, "molecular imaging" is represented by F, "mammography" is represented by G, "imaging Informatics" is represented by H.

### Radiologic Technologist:

The "radiologic technologist" or 'radiographer' is a specially trained healthcare professional that uses the imaging technology and positioning techniques to produce medical images for the radiologist to interpret. Depending on the individual's training and country of practice, the radiographer may specialize in one imaging modality or have expanded roles in image reporting. Here, the radiological technologist can be referred to as a radiology technologist or simply technologist. And as detailed, such technologist can specialise in specific imaging modalities.

### Interventional Radiologists:

Interventional radiologists are doctors who diagnose and treat patients using image-guided, minimally invasive techniques such as X-rays, MRI and fluoroscopy.

### Teleradiology

Teleradiology is the transmission of radiological patient images, such as x-rays, CTs, and MRIs, from one location to another for the purposes of sharing studies with other radiologists and physicians. Teleradiology allows radiologists to provide services without actually having to be at the location of the patient. This is particularly important when a sub-specialist such as an MRI radiologist, neuroradiologist, paediatric radiologist, or musculoskeletal radiologist is needed, since these professionals are generally only located in large metropolitan areas working during daytime hours. Teleradiology allows for trained specialists to be available 24/7.

Teleradiology utilizes standard network technologies such as the internet, telephone lines, wide area network, local area network (LAN) and the latest high tech being computer clouds. Specialized software is used to transmit the images and enable the radiologist to effectively analyse what can be hundreds of images for a given study. Technologies such as advanced graphics processing, voice recognition, artificial intelligence and image compression are often used in teleradiology. Through teleradiology and mobile DICOM viewers, images can be sent to another part of the hospital, or to other locations around the world.

For some Teleradiology services, the turnaround time (preliminary Reports include all pertinent findings and a phone call for any critical findings) is rapid with a 30-minute standard turnaround and expedited for critical and stroke studies. In the United States, Medicare and Medicaid laws require the teleradiologist to be on U.S. soil in order to qualify for reimbursement of the Final Read. In addition, advanced teleradiology systems must also be HIPAA compliant, which helps to ensure patients' privacy. HIPAA (Health Insurance Portability and Accountability Act of 1996) is a uniform, federal floor of privacy protections for consumers. It limits the ways that entities can use patients' personal information and protects the privacy of all medical information no matter what form it is in. Quality teleradiology must abide by important HIPAA rules to ensure patients' privacy is protected.

For more details see: https://en.wikipedia.org/wiki/Teleradiology.

### DICOM

Digital Imaging and Communications in Medicine (DICOM) is the standard for the communication and management of medical imaging information and related data. DICOM is most commonly used for storing and transmitting medical images enabling the integration of medical imaging devices such as scanners, servers, workstations, printers, network hardware, and picture archiving and communication systems (PACS) from multiple manufacturers. It has been widely adopted by hospitals and is making inroads into smaller applications such as dentists' and doctors' offices.

DICOM incorporates standards for imaging modalities such as radiography, ultrasonography, computed tomography (CT), magnetic resonance imaging (MRI), and radiation therapy. DICOM includes protocols for image exchange (e.g., via portable media such as DVDs), image compression, 3-D visualization, image presentation, and results reporting. The DICOM Store service is used to send images or other persistent objects (structured reports, etc.) to a picture archiving and communication system (PACS) or workstation.

For more details see: https://en.wikipedia.org/wiki/DICOM.

### Radiologist Efficiency

The average emergency department (ED) report turnaround time has steadily dropped across all modalities. This is largely due to technological advances, including voice recognition software and image-sharing platforms. Research shows that radiologists experience distractions and additional tasks that interfere with reading up to five times per hour, many of which are unrelated to patient care and/or are unnecessary.

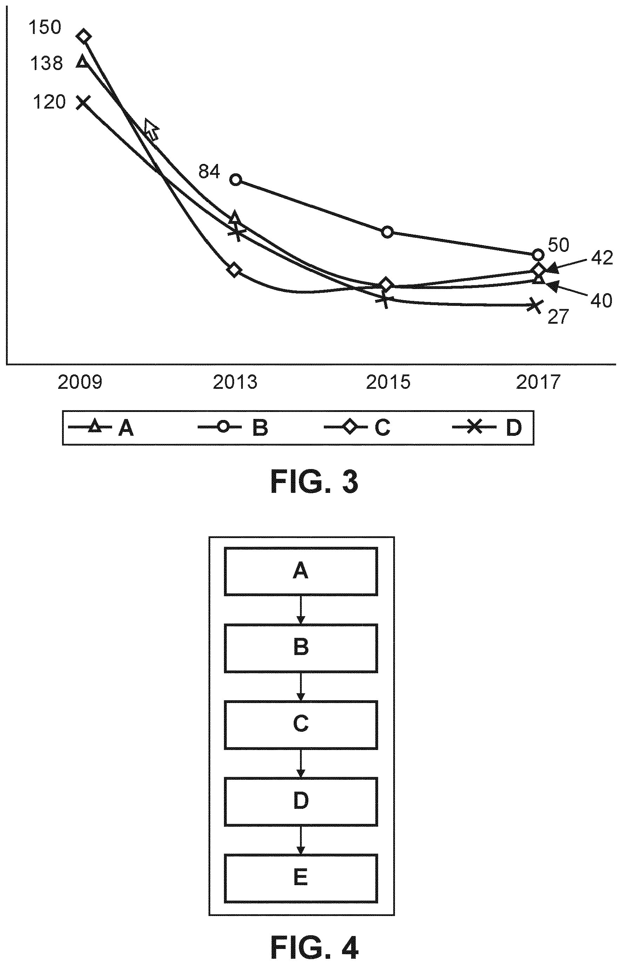

Fig. 3 shows the average ED report turnaround time and how it has changed with time. In Fig. 3 "CT" is indicated by A, "MRI" is indicated by B, "ultrasound" is indicated by C, and "x-ray" is indicated by D.

Fig. 4 shows the present standard workflow in radiology, where "referring physician (write imaging request)" is indicated by A, "diagnostic radiologists/radiology lab (imaging type in settings)" is indicated by B, "radiological technologist (perform imaging and send images)" is indicated by C, "diagnostic radiologists (read images and write report)" is indicated by D, and "referring physician (read report and review images)" is indicated by E.

Thus, the standard radiology workflow can be summarised as:
a referring physician creates an imaging request for a patient, in an electronic system or on paper
the request is received by a radiology lab (selected by the patient, or in the same hospital), and the patient is planned in for an exam
the exam is executed by the radiologic technician, according to the request, who sends the images to the radiologist
the radiologist reads the images and writes a report
the report is sent to the referring physician for review

This workflow suffers from inefficiencies caused by incomplete or unclear imaging requests, which result in (unnecessary) clarification phone calls or messages between the Referring Physician ordering the imaging, and both the Technician Radiologist (performing the imaging) and the Diagnostic Radiologists reading and diagnosing the imaging. And in the worst-case patients need to come back for a re-exam.

To counter the problems above, several systems have been proposed to structure and formalize image request creation to assure completeness and clearness, as well as protocol adherence. One such solution relates to the filling out of forms.

However, the current method of imaging requests is not well accepted by the profession. Diagnostic Imaging request creation using form-filling systems are considered too:
Time consuming
Restrictive
Complex
Not intuitive/user friendly

Form filling is not welcomed by the profession, who feel their time is better spent doing other activities. As a result, the imaging orders are still often rushed resulting in being incomplete, incorrect, and/or lacking relevant clinical context. This results in:
Incorrect imaging studies performed
Unnecessary imaging studies performed
Follow-up calls/faxes etc between the requesting physician and radiology for clarification
Radiologists spending unproductive time searching for clinical context

### Radiologists using their best guess to fill in the blanks

As the current method of ordering imaging is frustrating and time consuming, this has led to physicians 'tricking the system' to get the desired imaging study (e.g. always stating abdominal pain, when they want an abdominal CT). This also does not contribute to consistency of care.

More detail on the form filling solution can be found at:
https://www.sciencedirect.com/science/article/pii/S1546144011005928.

There is a need to resolve these issues.

### SUMMARY OF THE INVENTION

It would be advantageous to provide an improved technique to facilitate the whole medical imaging processing process from the requesting of medical imagery by a physician, to the setting up and carrying out a medical imaging examination on a medical imaging system (for example, attenuation X-ray, CT X-ray, Magnetic Resonance, positron emission tomography, ultrasound) by a technician (i.e. a radiology technician), to the assessment of the imagery and writing of a report by a medical expert (i.e. a radiologist).

The object of the present invention is solved with the subject matter of the independent claims, wherein further embodiments are incorporated in the dependent claims. It should be noted that the following described aspects and examples of the invention apply to the medical imaging processing systems, the medical imaging processing methods, as well as to a computer program element and a computer readable medium,

In a first aspect, there is provided a medical imaging processing system, comprising:
- a first virtual assistant;
- a second virtual assistant; and
- a third virtual assistant.

The first virtual assistant is configured to support a physician to prepare an imaging request for a patient. The imaging request is to be used by a technician who will carry out an imaging exam of the patient to generate patient imagery. The first virtual assistant is configured to utilize one or more of the following to support the physician to prepare the imaging request:
- information on the patient,
- information from a medical database,
- information on the physician,
- information on previous imaging requests,
- the physician's response to dialogue with the first virtual assistant, wherein the dialogue with the first virtual assistant comprises utilization of a VDU and/or a chatbot implemented by the first virtual assistant;

The second virtual assistant is configured to support the technician to carry out the imaging exam of the patient according to the imaging request to generate the patient imagery. The second virtual assistant is configured to utilize one or more of the following to support the technician to carry out the imaging exam of the patient according to the imaging request:
- information on the patient,
- information on the technician,
- information on a medical expert who will review the patient imagery and generate a report for the physician; and

The third virtual assistant is configured to support the medical expert to review the patient imagery and generate the report for the physician. The third virtual assistant is configured to utilize one or more of the following to support the medical expert to review the patient imagery and generate the report for the physician:
- information on the patient,
- information on the medical expert,
- information on previous reports generated from patient imagery,
- the medical expert's response to dialogue with the third virtual assistant, wherein the dialogue with the third virtual assistant comprises utilization of a VDU and/or a chatbot implemented by the third virtual assistant.

In an example, the first virtual assistant is configured to receive input from an eye tracking apparatus and/or from a microphone and/or from a camera. The physician's response to dialogue with the first virtual assistant comprises assessment of the physician's gaze direction with respect to content presented on the VDU and/or assessment of the physician's verbal response to content presented via the chatbot and/or assessment of the physician's facial expression response to the dialogue.

Thus, as the person interacts with the virtual assistant in a dialogue an assessment can be made as to what the person is looking at, and/or how they speak, and/or how their facial expression reacts to the dialogue, and this can inform what they consider to be important, whether they sound or look confused or in doubt, or whether they are certain about particular points raised during the dialogue.

In an example, the first virtual assistant is configured to receive written and/or verbal correspondence between the physician and the technician. The first virtual assistant is configured to utilize the written and/or verbal correspondence between the physician and the technician to support the physician to prepare the imaging request.

In an example, the first virtual assistant is configured to receive written and/or verbal correspondence between the physician and the medical expert and the first virtual assistant is configured to utilize the written and/or verbal correspondence between the physician and the medical expert to support the physician to prepare the imaging request.

In an example, the second virtual assistant is configured to utilize the following to support the technician to carry out the imaging exam of the patient according to the imaging request:
- the technician's response to dialogue with the second virtual assistant, wherein the dialogue with the second virtual assistant comprises utilization of a VDU and/or a chatbot implemented by the second virtual assistant.

In an example, the second virtual assistant is configured to receive written and/or verbal correspondence between the technician and the physician and the second virtual assistant is configured to utilize the written and/or verbal correspondence between the technician and the physician to support the technician to carry out the imaging exam of the patient according to the imaging request.

In an example, the second virtual assistant is configured to receive written and/or verbal correspondence between the technician and the medical expert and the second virtual assistant is configured to utilize the written and/or verbal correspondence between the technician and the medical expert to support the technician to carry out the imaging exam of the patient according to the imaging request.

In an example, the third virtual assistant is configured to receive input from an eye tracking apparatus and/or from a microphone and/or from a camera. The medical expert's response to dialogue with the third virtual assistant comprises assessment of the medical expert's gaze direction with respect to content presented on the VDU and/or assessment of the medical expert's verbal response to content presented via the chatbot and/or assessment of the medical expert's facial expression response to the dialogue.

In an example, the third virtual assistant is configured to receive written and/or verbal correspondence between the medical expert and the physician and the third virtual assistant is configured to utilize the written and/or verbal correspondence between the medical expert and the physician to support the medical expert to review the patient imagery and generate a report for the physician.

In an example, the third virtual assistant is configured to receive written and/or verbal correspondence between the medical expert and the technician and wherein the third virtual assistant is configured to utilize the written and/or verbal correspondence between the medical expert and the technician to support the medical expert to review the patient imagery and generate a report for the physician.

In an example, based on the support provided to the physician to prepare the imaging request the first virtual assistant is configured to update the information on the physician and/or to update the information on the technician and/or to update the information on the medical expert.

In an example, based on the support provided to the technician to carry out the imaging exam of the patient according to the imaging request the second virtual assistant is configured to update the information on the physician and/or to update the information on the technician and/or to update the information on the medical expert.

In an example, based on the support provided to the medical expert to review the patient imagery and generate the report for the physician the third virtual assistant is configured to update the information on the physician and/or to update the information on the technician and/or to update the information on the medical expert.

Thus, information on a person stored for example in a database can be updated, where that information could relates to any points of strength or weakness in a person's technical skill set, that enables the virtual assistant to ensure that appropriate support is provided, whether the person prefers to work in a certain way and that is indeed efficient for that person, whether they have made errors or conversely made important points regarding the imaging request that have led to an overall efficient previous process. These are examples of the information of person's that can be updated, enabling one or more virtual assistants to efficiently and effectively support the whole process.

In a second aspect, there is provided a medical imaging processing method, comprising: supporting by a first virtual assistant a physician to prepare an imaging request for a patient, wherein the imaging request is to be used by a technician who will carry out an imaging exam of the patient to generate patient imagery, and wherein the first virtual assistant utilizes one or more of the following to support the physician to prepare the imaging request:
- information on the patient,
- information from a medical database,
- information on the physician,
- information on previous imaging requests,
- the physician's response to dialogue with the first virtual assistant, wherein the dialogue with the first virtual assistant comprises utilization of a VDU and/or a chatbot implemented by the first virtual assistant;
supporting by a second virtual assistant the technician to carry out the imaging exam of the patient according to the imaging request to generate the patient imagery, and wherein the second virtual assistant utilizes one or more of the following to support the technician to carry out the imaging exam of the patient according to the imaging request:
- information on the patient,
- information on the technician,
- information on a medical expert who will review the patient imagery and generate a report for the physician; and
supporting by a third virtual assistant the medical expert to review the patient imagery and generate the report for the physician, and wherein the third virtual assistant utilizes one or more of the following to support the medical expert to review the patient imagery and generate the report for the physician:
- information on the patient,
- information on the medical expert,
- information on previous reports generated from patient imagery,
- the medical expert's response to dialogue with the third virtual assistant, wherein the dialogue with the third virtual assistant comprises utilization of a VDU and/or a chatbot implemented by the third virtual assistant.

In a third aspect, there is provided a medical imaging processing system, comprising:
- a first virtual assistant; and
- a second virtual assistant.

The first virtual assistant is configured to support a physician to prepare an imaging request for a patient. The imaging request is to be used by a technician who will carry out an imaging exam of the patient to generate patient imagery. The first virtual assistant is configured to utilize one or more of the following to support the physician to prepare the imaging request:
- information on the patient,
- information from a medical database,
- information on the physician,
- information on previous imaging requests,
- the physician's response to dialogue with the first virtual assistant, wherein the dialogue with the first virtual assistant comprises utilization of a VDU and/or a chatbot implemented by the first virtual assistant;

The second virtual assistant is configured to support the technician to carry out the imaging exam of the patient according to the imaging request to generate the patient imagery. The second virtual assistant is configured to utilize one or more of the following to support the technician to carry out the imaging exam of the patient according to the imaging request:
- information on the patient
- information on the technician,
- information on a medical expert who will review the patient imagery and generate a report for the physician; and

The patient imagery is to be provided to the medical expert to review and generate the report for the physician.

In a fourth aspect, there is provided a medical imaging processing method, comprising:
supporting by a first virtual assistant a physician to prepare an imaging request for a patient, wherein the imaging request is to be used by a technician who will carry out an imaging exam of the patient to generate patient imagery. The first virtual assistant utilizes one or more of the following to support the physician to prepare the imaging request:
- information on the patient,
- information from a medical database,
- information on the physician,
- information on previous imaging requests,
- the physician's response to dialogue with the first virtual assistant, wherein the dialogue with the first virtual assistant comprises utilization of a VDU and/or a chatbot implemented by the first virtual assistant;
- supporting by a second virtual assistant the technician to carry out the imaging exam of the patient according to the imaging request to generate the patient imagery. The second virtual assistant utilizes one or more of the following to support the technician to carry out the imaging exam of the patient according to the imaging request:
- information on the patient,
- information on the technician,
- information on a medical expert who will review the patient imagery and generate a report for the physician; and
The patient imagery is provided to the medical expert to review and generate the report for the physician.

In a fifth aspect, there is provided a medical imaging processing system, comprising:
- a first virtual assistant; and
- a second virtual assistant.

The first virtual assistant is configured to support a physician to prepare an imaging request for a patient. The imaging request is to be used by a technician who will carry out an imaging exam of the patient to generate patient imagery. The first virtual assistant is configured to utilize one or more of the following to support the physician to prepare the imaging request:
- information on the patient,
- information from a medical database,
- information on the physician,
- information on previous imaging requests,
- the physician's response to dialogue with the first virtual assistant, wherein the dialogue with the first virtual assistant comprises utilization of a VDU and/or a chatbot implemented by the first virtual assistant.

The imaging request is to be provided to the technician to carry out the imaging exam of the patient according to the imaging request to generate the patient imagery. The second virtual assistant is configured to support the medical expert to review the patient imagery and generate the report for the physician. The second virtual assistant is configured to utilize one or more of the following to support the medical expert to review the patient imagery and generate the report for the physician:
- information on the patient,
- information on the medical expert,
- information on previous reports generated from patient imagery,
- the medical expert's response to dialogue with the second virtual assistant, wherein the dialogue with the second virtual assistant comprises utilization of a VDU and/or a chatbot implemented by the first virtual assistant.

In a sixth aspect, there is provided a medical imaging processing method, comprising:
supporting by a first virtual assistant a physician to prepare an imaging request for a patient, wherein the imaging request is to be used by a technician who will carry out an imaging exam of the patient to generate patient imagery, and wherein the first virtual assistant utilizes one or more of the following to support the physician to prepare the imaging request:
- information on the patient,
- information from a medical database,
- information on the physician,
- information on previous imaging requests,
- the physician's response to dialogue with the first virtual assistant, wherein the dialogue with the first virtual assistant comprises utilization of a VDU and/or a chatbot implemented by the first virtual assistant;
providing the imaging request to the technician to carry out the imaging exam of the patient according to the imaging request to generate the patient imagery; and
supporting by a second virtual assistant the medical expert to review the patient imagery and generate the report for the physician, and wherein the second virtual assistant utilizes one or more of the following to support the medical expert to review the patient imagery and generate the report for the physician:
- information on the patient,
- information on the medical expert,
- information on previous reports generated from patient imagery,
- the medical expert's response to dialogue with the second virtual assistant, wherein the dialogue with the second virtual assistant comprises utilization of a VDU and/or a chatbot implemented by the second virtual assistant.

In a seventh aspect, there is provided a medical imaging processing system, comprising:
- a first virtual assistant; and
- a second virtual assistant.
The first virtual assistant is configured to support a technician to carry out an imaging exam of a patient according to an imaging request prepared by a physician to generate patient imagery. The first virtual assistant is configured to utilize one or more of the following to support the technician to carry out the imaging exam of the patient according to the imaging request:
- information on the patient,
- information on the technician,
- information on a medical expert who will review the patient imagery and generate a report for the physician.

The second virtual assistant is configured to support the medical expert to review the patient imagery and generate the report for the physician. The second virtual assistant is configured to utilize one or more of the following to support the medical expert to review the patient imagery and generate the report for the physician:
- information on the patient,
- information on the medical expert,
- information on previous reports generated from patient imagery,
- the medical expert's response to dialogue with the third virtual assistant, wherein the dialogue with the third virtual assistant comprises utilization of a VDU and/or a chatbot implemented by the third virtual assistant.

In an eighth aspect, there is provided a medical imaging processing method, comprising:
supporting by a first virtual assistant a technician to carry out an imaging exam of a patient according to an imaging request prepared by a physician to generate patient imagery, and wherein the first virtual assistant utilizes one or more of the following to support the technician to carry out the imaging exam of the patient according to the imaging request:
   - information on the patient,
   - information on the technician,
   - information on a medical expert who will review the patient imagery and generate a report for the physician; and
supporting by a second virtual assistant the medical expert to review the patient imagery and generate the report for the physician, and wherein the second virtual assistant utilizes one or more of the following to support the medical expert to review the patient imagery and generate the report for the physician:
   - information on the patient,
   - information on the medical expert,
   - information on previous reports generated from patient imagery,
   - the medical expert's response to dialogue with the third virtual assistant, wherein the dialogue with the third virtual assistant comprises utilization of a VDU and/or a chatbot implemented by the third virtual assistant.

According to aspects, there is provided computer program elements controlling one or more of the systems as previously described which, if the computer program element is executed by a processor, is adapted to perform one or more of the associated methods as previously described.

According to other aspects, there is provided computer readable media having stored the computer elements as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a representation of a diagnostic radiologist "reading" images to diagnose and document;
Fig. 2 shows a representation of medical imaging Informatics global market;
Fig. 3 shows a representation of the average emergency department report turnaround time;
Fig. 4 shows a representation of the current or standard radiology workflow;
Fig. 5 shows a schematic representation of an example of the newly developed medical imaging processing system;
Fig. 6 shows an example of the newly developed medical imaging processing method
Fig. 7 shows a schematic representation of a detailed example of the newly developed medical imaging processing system;
Fig. 8 shows a schematic representation of a detailed example of a subset of the newly developed medical imaging processing system; and
Fig. 9 shows a schematic representation of a detailed example of the newly developed medical imaging processing system.

### DETAILED DESCRIPTION OF EMBODIMENTS

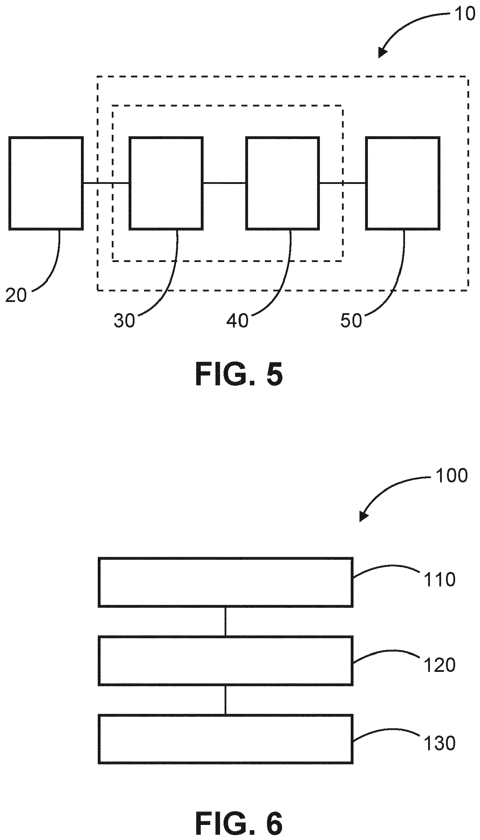

Fig. 5 shows a medical imaging processing system 10 comprising: a first virtual assistant 20, a second virtual assistant 30, 40, and a third virtual assistant 50. The first virtual assistant is configured to support a physician to prepare an imaging request for a patient. The imaging request is to be used by a technician who will carry out an imaging exam of the patient to generate patient imagery. The first virtual assistant is configured to utilize one or more of the following to support the physician to prepare the imaging request: information on the patient, information from a medical database, information on the physician, information on previous imaging requests, the physician's response to dialogue with the first virtual assistant, and where the dialogue with the first virtual assistant comprises utilization of a VDU and/or a chatbot implemented by the first virtual assistant. The second virtual assistant is configured to support the technician to carry out the imaging exam of the patient according to the imaging request to generate the patient imagery. The second virtual assistant is configured to utilize one or more of the following to support the technician to carry out the imaging exam of the patient according to the imaging request: information on the patient, information on the technician, information on a medical expert who will review the patient imagery and generate a report for the physician. The third virtual assistant is configured to support the medical expert to review the patient imagery and generate the report for the physician. The third virtual assistant is configured to utilize one or more of the following to support the medical expert to review the patient imagery and generate the report for the physician: information on the patient, information on the medical expert, information on previous reports generated from patient imagery, the medical expert's response to dialogue with the third virtual assistant, wherein the dialogue with the third virtual assistant comprises utilization of a VDU and/or a chatbot implemented by the third virtual assistant.

In an example, the technician is a radiology technician who sets up and carries out an X-ray exam, that could be an attenuation exam such as a mammogram or an X-ray CT exam.

In an example, the medical expert is a radiologist. In an example, the technician is an MRI technician who sets up and carries out an MRI exam. In an example, the medical expert is an MRI medical expert. In an example, the technician is a PET technician who sets up and carries out a PET exam. In an example, the medical expert is a PET medical expert. In an example, the technician is an ultrasound technician who sets up and carries out an ultrasound exam. In an example, the medical expert is an ultrasound medical expert.

In an example, the second virtual assistant can be considered to be two virtual assistants; a first that supports the radiology technician to set up the exam and a second that supports the radiology technician to carry out the exam.

In general comments are made with respect to a radiology technician and a radiologist, but this applies to all medical imaging technicians who set up and carry out an exam and all medical experts who review the imagery and write a report.

In an example, the second virtual assistant and the third virtual assistant are implemented as the same virtual assistant.

Thus, for example in a hospital where a radiology technician and a radiologist work, the same virtual assistant can support both the radiology technician and the radiologist - the same applies for MRI, PET, ultrasound or any other medical imaging process. The second and third virtual assistant can however be different.

In an example, the first virtual assistant, the second virtual assistant and the third virtual assistant are implemented as the same virtual assistant.

Thus, for example in a hospital where a physician works, the physician can interact with a patient being supported by a virtual assistant to prepare an imaging request. A radiology technician and a radiologist can also work in same hospital, where the same virtual assistant that supported the physician can also support both the radiology technician and the radiologist - the same applies for MRI, PET, ultrasound or any other medical imaging process. The first, second and third virtual assistants can however be different.

Thus, the second and the third virtual assistants, whether implemented as the same virtual assistant or as separate virtual assistants can operate simultaneously. For example, the radiology technician could telephone the radiologist or send the radiologist an email regarding the exam they are to perform to ask for feedback. The second virtual assistant can monitor this correspondence and take it into account when supporting the radiology technician prepare for and take the exam. For example the second virtual assistant can monitor what the radiology technician says and whether their language or the way they speak, with pauses or hesitancy, for example shows uncertainty on certain points. For example the second virtual assistant can monitor the radiology technician's gaze, where they may be looking at a particular section of the imaging request during the call with the radiologist indicating that the radiology technician considered something to be particularly relevant, in terms of not being clear, or not being understood or even a key aspect of the exam to be carried out. The second virtual assistant can use this in supporting the radiology technician and also on the basis of the information derived in this way update information on the radiology technician and on the radiologist and even on the physician, if for example the imaging request was not clear on a point, the radiology technician misunderstood something or had a lack of experience on a point or noticed something of key relevance, or the radiologist preferred certain types of imagery. Thus, not only can the second virtual assistant provide support for the radiology technician but information utilized by the second and third virtual assistants is updated enabling these to operate ever for efficiently. The third virtual assistant can also monitor the correspondence between the radiology technician and the radiologist in the same way as discussed above, where during that correspondence the radiologist speech can also be monitored and what they are looking at - for example they may also pull up a copy of the imaging request during the call, and/or copies of similar imaging requests, and the radiologists gaze can also be tracked to assess what they consider to be important. This information can then be used to support the radiologist and update information on the physician, radiology technician, and even physician.

Also, the first, second and the third virtual assistants, whether implemented as the same virtual assistant or as separate virtual assistants can operate simultaneously. For example, the physician could telephone both the radiology technician and the radiologist, or send emails to both, regarding the imaging request and asking for feedback. Each of the first, second and third virtual assistants can monitor this correspondence and use this in supporting the relevant person. Also, the information on each of the physician, the radiology technician and the radiologist can be updated based on these interactions.

This process of monitoring the physician, radiology technician and the radiologist can also take place when they are not corresponding with each other, but when they are working on their associated tasks, where the virtual assistants enter dialogue with the relevant person, for example asking questions about performance of the relevant task, and monitoring of how the relevant person responds via speech monitoring and/or gaze monitoring the relevant assistant can support the relevant person, and also update the information on each of the physician, radiology technician and the radiologist.

This monitoring process also applies to the physician when preparing the imaging request, monitoring dialogue with the virtual assistant and correspondence with the radiology technician and/or radiologist, and in addition to supporting the physician as detailed above, the information on the physician, the radiology technician and the radiologist can be updated, thereby providing a feedback loop of continual improvement of support across the system.

In an example, the first virtual assistant is configured to utilize one or more of the following to support the physician to prepare the imaging request: information on the technician who will carry out the imaging exam of the patient according to the imaging request to generate the patient imagery, information on previous exams carried out on the basis of previous imaging requests, information on the medical expert who will review the patient imagery and generate the report for the physician, information on previous reports generated from patient imagery acquired in accordance with previous imaging requests.

According to an example, the first virtual assistant is configured to receive input from an eye tracking apparatus and/or from a microphone and/or from a camera. The physician's response to dialogue with the first virtual assistant comprises assessment of the physician's gaze direction with respect to content presented on the VDU and/or assessment of the physician's verbal response to content presented via the chatbot and/or assessment of the physician's facial expression response to the dialogue.

Thus, as the person interacts with the virtual assistant in a dialogue an assessment can be made as to what the person is looking at, and/or how they speak, and/or how their facial expression reacts to the dialogue, and this can inform what they consider to be important, whether they sound or look confused or in doubt, or whether they are certain about particular points raised during the dialogue.

According to an example, the first virtual assistant is configured to receive written and/or verbal correspondence between the physician and the technician and the first virtual assistant is configured to utilize the written and/or verbal correspondence between the physician and the technician to support the physician to prepare the imaging request. Additionally or alternatively the first virtual assistant is configured to receive written and/or verbal correspondence between the physician and the medical expert and the first virtual assistant is configured to utilize the written and/or verbal correspondence between the physician and the medical expert to support the physician to prepare the imaging request.

In an example, the second virtual assistant is configured to utilize one or more of the following to support the technician to carry out the imaging exam of the patient according to the imaging request: information from the medical database, information on the physician, information on previous imaging requests, information on previous exams carried out on the basis of previous imaging requests, information on previous reports generated from patient imagery generated from previous exams carried out on the basis of previous imaging requests.

According to an example, the second virtual assistant is configured to utilize the following to support the technician to carry out the imaging exam of the patient according to the imaging request: the technician's response to dialogue with the second virtual assistant, wherein the dialogue with the second virtual assistant comprises utilization of a VDU and/or a chatbot implemented by the second virtual assistant.

In an example, the second virtual assistant is configured to receive input from an eye tracking apparatus and/or from a microphone and/or from a camera. The technician's response to dialogue with the second virtual assistant comprises assessment of the technician's gaze direction with respect to content presented on the VDU and/or assessment of the technician's verbal response to content presented via the chatbot and/or assessment of the technician's facial expression response to the dialogue.

According to an example, the second virtual assistant is configured to receive written and/or verbal correspondence between the technician and the physician and the second virtual assistant is configured to utilize the written and/or verbal correspondence between the technician and the physician to support the technician to carry out the imaging exam of the patient according to the imaging request. Additionally or alternatively the second virtual assistant is configured to receive written and/or verbal correspondence between the technician and the medical expert and wherein the second virtual assistant is configured to utilize the written and/or verbal correspondence between the technician and the medical expert to support the technician to carry out the imaging exam of the patient according to the imaging request.

According to an example, the third virtual assistant is configured to receive input from an eye tracking apparatus and/or from a microphone and/or from a camera, and the medical expert's response to dialogue with the third virtual assistant comprises assessment of the medical expert's gaze direction with respect to content presented on the VDU and/or assessment of the medical expert's verbal response to content presented via the chatbot and/or assessment of the medical expert's facial expression response to the dialogue.

In an example, the third virtual assistant is configured to utilize one or more of the following to support the medical expert to review the patient imagery and generate the report for the physician: information on the physician, information on the technician, information from the medical database.

According to an example, the third virtual assistant is configured to receive written and/or verbal correspondence between the medical expert and the physician and the third virtual assistant is configured to utilize the written and/or verbal correspondence between the medical expert and the physician to support the medical expert to review the patient imagery and generate a report for the physician. Additionally or alternatively the third virtual assistant is configured to receive written and/or verbal correspondence between the medical expert and the technician and the third virtual assistant is configured to utilize the written and/or verbal correspondence between the medical expert and the technician to support the medical expert to review the patient imagery and generate a report for the physician.

According to an example, based on the support provided to the physician to prepare the imaging request the first virtual assistant is configured to update the information on the physician and/or to update the information on the technician and/or to update the information on the medical expert. Additionally or alternatively based on the support provided to the technician to carry out the imaging exam of the patient according to the imaging request the second virtual assistant is configured to update the information on the physician and/or to update the information on the technician and/or to update the information on the medical expert. Additionally or alternatively based on the support provided to the medical expert to review the patient imagery and generate the report for the physician the third virtual assistant is configured to update the information on the physician and/or to update the information on the technician and/or to update the information on the medical expert.

Thus, information on a person stored for example in a database can be updated, where that information could relates to any points of strength or weakness in a person's technical skill set, that enables the virtual assistant to ensure that appropriate support is provided, whether the person prefers to work in a certain way and that is indeed efficient for that person, whether they have made errors or conversely made important points regarding the imaging request that have led to an overall efficient previous process. These are examples of the information of person's that can be updated, enabling one or more virtual assistants to efficiently and effectively support the whole process.

The system of Fig. 5 can however operate without one of the three parties receiving virtual assistant support. Such a medical imaging processing system 10 comprises a first virtual assistant 20, and a second virtual assistant 30, 40. The first virtual assistant is configured to support a physician to prepare an imaging request for a patient. The imaging request is to be used by a technician who will carry out an imaging exam of the patient to generate patient imagery. The first virtual assistant is configured to utilize one or more of the following to support the physician to prepare the imaging request: information on the patient, information from a medical database, information on the physician, information on previous imaging requests, the physician's response to dialogue with the first virtual assistant, wherein the dialogue with the first virtual assistant comprises utilization of a VDU and/or a chatbot implemented by the first virtual assistant. The second virtual assistant is configured to support the technician to carry out the imaging exam of the patient according to the imaging request to generate the patient imagery. The second virtual assistant is configured to utilize one or more of the following to support the technician to carry out the imaging exam of the patient according to the imaging request: information on the patient, information on the technician, information on a medical expert who will review the patient imagery and generate a report for the physician. The patient imagery is to be provided to the medical expert to review and generate the report for the physician.

In an example, the technician is a radiology technician who sets up and carries out an X-ray exam, that could be an attenuation exam such as a mammogram or an X-ray CT exam. In an example, the medical expert is a radiologist. In an example, the technician is an MRI technician who sets up and carries out an MRI exam. In an example, the medical expert is an MRI medical expert. In an example, the technician is a PET technician who sets up and carries out a PET exam. In an example, the medical expert is a PET medical expert. In an example, the technician is an ultrasound technician who sets up and carries out an ultrasound exam. In an example, the medical expert is an ultrasound medical expert.

In an example, the second virtual assistant can be considered to be two virtual assistants; a first that supports the radiology technician to set up the exam and a second that supports the radiology technician to carry out the exam.

In general comments are made with respect to a radiology technician and a radiologist, but this applies to all medical imaging technicians who set up and carry out an exam and all medical experts who review the imagery and write a report.

In an example, the first virtual assistant and the second virtual assistant are implemented as the same virtual assistant.

Thus, for example in a hospital where a physician and a radiology technician work, the same virtual assistant can support both the physician to prepare an imaging request and radiology technician to set up and carry out the exam. The first and second virtual assistants can however be different.

However, the first and the second virtual assistants, whether implemented as the same virtual assistant or as separate virtual assistants can operate simultaneously. For example, the radiology technician could telephone the physician or send the physician an email regarding the exam they are to perform to ask for feedback. The second virtual assistant can monitor this correspondence and take it into account when supporting the radiology technician prepare for and take the exam. For example the second virtual assistant can monitor what the physician says and whether their language or the way they speak, with pauses or hesitancy, for example shows uncertainty on certain points. For example the second virtual assistant can monitor the radiology technician's gaze, where they may be looking at a particular section of the imaging request during the call with the physician indicating that the radiology technician considered something to be particularly relevant, in terms of not being clear, or not being understood or even a key aspect of the exam to be carried out. The second virtual assistant can use this in supporting the radiology technician and also on the basis of the information derived in this way update information on the radiology technician and on the physician and even on the radiologist, if for example the imaging request was not clear on a point, the radiology technician misunderstood something or had a lack of experience on a point or noticed something of key relevance. Thus, not only can the second virtual assistant provide support for the radiology technician but information utilized by the first and second virtual assistants is updated enabling these to operate ever for efficiently. The second virtual assistant can also monitor the correspondence between the radiology technician and the physician in the same way as discussed above, where during that correspondence the physician's speech can also be monitored and what they are looking at - for example they may also pull up a copy of the imaging request during the call, and/or copies of similar imaging requests, and the physician's gaze can also be tracked to assess what they consider to be important. This information can then be used to support the physician in the future by updating information on the physician, the radiology technician, and even the radiologist.

This process of monitoring the radiology technician and the physician can also take place when they are not corresponding with each other, but when they are working on their associated tasks, where the virtual assistants enter dialogue with the relevant person, for example asking questions about performance of the relevant task, and monitoring of how the relevant person responds via speech monitoring and/or gaze monitoring the relevant assistant can support the relevant person, and also update the information on each of the physician, radiology technician and the radiologist.

In an example, the first virtual assistant is configured to utilize one or more of the following to support the physician to prepare the imaging request: information on the technician who will carry out the imaging exam of the patient according to the imaging request to generate the patient imagery, information on previous exams carried out on the basis of previous imaging requests, information on the medical expert who will review the patient imagery and generate the report for the physician, information on previous reports generated from patient imagery acquired in accordance with previous imaging requests.

In an example, the first virtual assistant is configured to receive input from an eye tracking apparatus and/or from a microphone and/or camera, and the physician's response to dialogue with the first virtual assistant comprises assessment of the physician's gaze direction with respect to content presented on the VDU and/or assessment of the physician's verbal response to content presented via the chatbot and/or assessment of the physician's facial expression response to the dialogue.

In an example, the first virtual assistant is configured to receive written and/or verbal correspondence between the physician and the technician and the first virtual assistant is configured to utilize the written and/or verbal correspondence between the physician and the technician to support the physician to prepare the imaging request. Additionally or alternatively the first virtual assistant is configured to receive written and/or verbal correspondence between the physician and the medical expert and the first virtual assistant is configured to utilize the written and/or verbal correspondence between the physician and the medical expert to support the physician to prepare the imaging request.

In an example, the second virtual assistant is configured to utilize one or more of the following to support the technician to carry out the imaging exam of the patient according to the imaging request: information from the medical database, information on the physician, information on previous imaging requests, information on previous exams carried out on the basis of previous imaging requests, information on previous reports generated from patient imagery generated from previous exams carried out on the basis of previous imaging requests.

In an example, the second virtual assistant is configured to utilize the following to support the technician to carry out the imaging exam of the patient according to the imaging request: the technician's response to dialogue with the second virtual assistant, wherein the dialogue with the second virtual assistant comprises utilization of a VDU and/or a chatbot implemented by the second virtual assistant.

In an example, the second virtual assistant is configured to receive input from an eye tracking apparatus and/or from a microphone and/or a camera, and wherein the technician's response to dialogue with the second virtual assistant comprises assessment of the technician's gaze direction with respect to content presented on the VDU and/or assessment of the technician's verbal response to content presented via the chatbot and/or assessment of the technician's facial expression response to the dialogue.

In an example, the second virtual assistant is configured to receive written and/or verbal correspondence between the technician and the physician and the second virtual assistant is configured to utilize the written and/or verbal correspondence between the technician and the physician to support the technician to carry out the imaging exam of the patient according to the imaging request. Additionally or alternatively the second virtual assistant is configured to receive written and/or verbal correspondence between the technician and the medical expert and wherein the second virtual assistant is configured to utilize the written and/or verbal correspondence between the technician and the medical expert to support the technician to carry out the imaging exam of the patient according to the imaging request.

In an example, based on the support provided to the physician to prepare the imaging request the first virtual assistant is configured to update the information on the physician and/or to update the information on the technician and/or to update the information on the medical expert. Additionally or alternatively based on the support provided to the technician to carry out the imaging exam of the patient according to the imaging request the second virtual assistant is configured to update the information on the physician and/or to update the information on the technician and/or to update the information on the medical expert.

As detailed above the system of Fig. 5 can operate without one of the three parties receiving virtual assistant support. Another such a medical imaging processing system 10 comprises a first virtual assistant 20, and a second virtual assistant 50. The first virtual assistant is configured to support a physician to prepare an imaging request for a patient. The imaging request is to be used by a technician who will carry out an imaging exam of the patient to generate patient imagery. The first virtual assistant is configured to utilize one or more of the following to support the physician to prepare the imaging request: information on the patient, information from a medical database, information on the physician, information on previous imaging requests, the physician's response to dialogue with the first virtual assistant, wherein the dialogue with the first virtual assistant comprises utilization of a VDU and/or a chatbot implemented by the first virtual assistant. The imaging request is to be provided to the technician to carry out the imaging exam of the patient according to the imaging request to generate the patient imagery. The second virtual assistant is configured to support the medical expert to review the patient imagery and generate the report for the physician. The second virtual assistant is configured to utilize one or more of the following to support the medical expert to review the patient imagery and generate the report for the physician: information on the patient, information on the medical expert, information on previous reports generated from patient imagery, the medical expert's response to dialogue with the second virtual assistant, wherein the dialogue with the second virtual assistant comprises utilization of a VDU and/or a chatbot implemented by the first virtual assistant.

In an example, the technician is a radiology technician who sets up and carries out an X-ray exam, that could be an attenuation exam such as a mammogram or an X-ray CT exam. In an example, the medical expert is a radiologist. In an example, the technician is an MRI technician who sets up and carries out an MRI exam. In an example, the medical expert is an MRI medical expert. In an example, the technician is a PET technician who sets up and carries out a PET exam. In an example, the medical expert is a PET medical expert. In an example, the technician is an ultrasound technician who sets up and carries out an ultrasound exam. In an example, the medical expert is an ultrasound medical expert.

In general comments are made with respect to a radiologist, but this applies to all medical experts who review the imagery and write a report.

In an example, the first virtual assistant and the second virtual assistant are implemented as the same virtual assistant.

Thus, for example in a hospital where a physician and a radiologist work, the same virtual assistant can support both the physician and the radiologist - the same applies for MRI, PET, ultrasound or any other medical imaging process. The first and second virtual assistants can however be different.

Thus, for example in a hospital where a physician works, the physician can interact with a patient being supported by a virtual assistant to prepare an imaging request. A radiology technician then sets up and acquires imagery, and a radiologist can also work in same hospital, where the same virtual assistant that supported the physician can also support and the radiologist - the same applies for MRI, PET, ultrasound or any other medical imaging process.

Thus, the first and the second virtual assistants, whether implemented as the same virtual assistant or as separate virtual assistants can operate simultaneously. For example, the physician could telephone the radiologist or send the radiologist an email regarding the imaging request they are preparing to ask for feedback, or the radiologist can telephone or send the physician an email to ask questions about the imagery acquired for this patient with respect to the imaging request and the report they are writing to ask for feedback. Both the first and second virtual assistants can monitor this correspondence and take it into account when supporting the radiologist to prepare the report and support the physician when preparing future imaging requests. For example the first virtual assistant can monitor what the physician says and whether their language or the way they speak, with pauses or hesitancy, for example shows uncertainty on certain points. For example the first virtual assistant can monitor the physicians' gaze, where they may be looking at a particular section of the imaging request during the call with the radiologist indicating that the physician considered something to be particularly relevant, in terms of not being clear, or not being understood or even a key aspect of the exam that was to be carried out. The first virtual assistant can use this in supporting the physician in preparing future imaging requests and also on the basis of the information derived in this way update information on the physician and on the radiologist and even on the radiology technician, if for example the imaging request was not clear on a point, or the radiologist preferred certain types of imagery. Thus, not only can the first virtual assistant provide support for the physician but information utilized by the first and second virtual assistants is updated enabling these to operate ever for efficiently. As discussed above, the second virtual assistant can also monitor the correspondence between the physician and the radiologist in the same way as discussed above, where during that correspondence the radiologist's speech can also be monitored and what they are looking at - for example they may also pull up a copy of the imaging request during the call, and/or copies of similar imaging requests, and the radiologists gaze can also be tracked to assess what they consider to be important. This information can then be used to support the radiologist and update information on the physician, the radiologist and even the radiology technician.

This process of monitoring the physician and the radiologist can also take place when they are not corresponding with each other, but when they are working on their associated tasks, where the virtual assistants enter dialogue with the relevant person, for example asking questions about performance of the relevant task, and monitoring of how the relevant person responds via speech monitoring and/or gaze monitoring the relevant assistant can support the relevant person, and also update the information on each of the physician, radiology technician and the radiologist.

In an example, the first virtual assistant is configured to utilize one or more of the following to support the physician to prepare the imaging request: information on the technician who will carry out the imaging exam of the patient according to the imaging request to generate the patient imagery, information on previous exams carried out on the basis of previous imaging requests, information on the medical expert who will review the patient imagery and generate the report for the physician, information on previous reports generated from patient imagery acquired in accordance with previous imaging requests.

In an example, the first virtual assistant is configured to receive input from an eye tracking apparatus and/or from a microphone and/or, and the physician's response to dialogue with the first virtual assistant comprises assessment of the physician's gaze direction with respect to content presented on the VDU and/or assessment of the physician's verbal response to content presented via the chatbot and/or assessment of the physician's facial expression response to the dialogue.

In an example, the first virtual assistant is configured to receive written and/or verbal correspondence between the physician and the technician and the first virtual assistant is configured to utilize the written and/or verbal correspondence between the physician and the technician to support the physician to prepare the imaging request. Additionally or alternatively the first virtual assistant is configured to receive written and/or verbal correspondence between the physician and the medical expert and the first virtual assistant is configured to utilize the written and/or verbal correspondence between the physician and the medical expert to support the physician to prepare the imaging request.

In an example, the second virtual assistant is configured to receive input from an eye tracking apparatus and/or from a microphone and/or camera. The medical expert's response to dialogue with the second virtual assistant comprises assessment of the medical expert's gaze direction with respect to content presented on the VDU and/or assessment of the medical expert's verbal response to content presented via the chatbot and/or assessment of the medical expert's facial expression response to the dialogue.

In an example, the second virtual assistant is configured to utilize one or more of the following to support the medical expert to review the patient imagery and generate the report for the physician: information on the physician, information on the technician, information from the medical database.

In an example, the second virtual assistant is configured to receive written and/or verbal correspondence between the medical expert and the physician and the second virtual assistant is configured to utilize the written and/or verbal correspondence between the medical expert and the physician to support the medical expert to review the patient imagery and generate a report for the physician. Additionally or alternatively the second virtual assistant is configured to receive written and/or verbal correspondence between the medical expert and the technician and wherein the second virtual assistant is configured to utilize the written and/or verbal correspondence between the medical expert and the technician to support the medical expert to review the patient imagery and generate a report for the physician.

In an example, based on the support provided to the physician to prepare the imaging request the first virtual assistant is configured to update the information on the physician and/or to update the information on the technician and/or to update the information on the medical expert. Additionally or alternatively based on the support provided to the medical expert to review the patient imagery and generate the report for the physician the second virtual assistant is configured to update the information on the physician and/or to update the information on the technician and/or to update the information on the medical expert.

As detailed above the system of Fig. 5 can operate without one of the three parties receiving virtual assistant support. Another such a medical imaging processing system 10 comprises a first virtual assistant 30, 40, and a second virtual assistant 50. The first virtual assistant is configured to support a technician to carry out an imaging exam of a patient according to an imaging request prepared by a physician to generate patient imagery. The first virtual assistant is configured to utilize one or more of the following to support the technician to carry out the imaging exam of the patient according to the imaging request: information on the patient, information on the technician, information on a medical expert who will review the patient imagery and generate a report for the physician. The second virtual assistant is configured to support the medical expert to review the patient imagery and generate the report for the physician. The second virtual assistant is configured to utilize one or more of the following to support the medical expert to review the patient imagery and generate the report for the physician: information on the patient, information on the medical expert, information on previous reports generated from patient imagery, the medical expert's response to dialogue with the third virtual assistant, wherein the dialogue with the third virtual assistant comprises utilization of a VDU and/or a chatbot implemented by the third virtual assistant.

In an example, the technician is a radiology technician who sets up and carries out an X-ray exam, that could be an attenuation exam such as a mammogram or an X-ray CT exam. In an example, the medical expert is a radiologist. In an example, the technician is an MRI technician who sets up and carries out an MRI exam. In an example, the medical expert is an MRI medical expert. In an example, the technician is a PET technician who sets up and carries out a PET exam. In an example, the medical expert is a PET medical expert. In an example, the technician is an ultrasound technician who sets up and carries out an ultrasound exam. In an example, the medical expert is an ultrasound medical expert.

In an example, the first virtual assistant can be considered to be two virtual assistants; a first that supports the radiology technician to set up the exam and a second that supports the radiology technician to carry out the exam.

In general comments are made with respect to a radiology technician and a radiologist, but this applies to all medical imaging technicians who set up and carry out an exam and all medical experts who review the imagery and write a report.

In an example, the first virtual assistant and the second virtual assistant are implemented as the same virtual assistant.

Thus, for example in a hospital where a radiology technician and a radiologist work, the same virtual assistant can support both the radiology technician and the radiologist - the same applies for MRI, PET, ultrasound or any other medical imaging process. The first and second virtual assistants can however be different.

The first and the second virtual assistants, whether implemented as the same virtual assistant or as separate virtual assistants can operate simultaneously or at different times. For example, the radiology technician could telephone the radiologist or send the radiologist an email regarding the exam they are to perform to ask for feedback. The first virtual assistant can monitor this correspondence and take it into account when supporting the radiology technician prepare for and take the exam. For example the first virtual assistant can monitor what the radiology technician says and whether their language or the way they speak, with pauses or hesitancy, for example shows uncertainty on certain points. For example the first virtual assistant can monitor the radiology technician's gaze, where they may be looking at a particular section of the imaging request during the call with the radiologist indicating that the radiology technician considered something to be particularly relevant, in terms of not being clear, or not being understood or even a key aspect of the exam to be carried out. The first virtual assistant can use this in supporting the radiology technician and also on the basis of the information derived in this way update information on the radiology technician and on the radiologist and even on the physician, if for example the imaging request was not clear on a point, the radiology technician misunderstood something or had a lack of experience on a point or noticed something of key relevance, or the radiologist preferred certain types of imagery. Thus, not only can the first virtual assistant provide support for the radiology technician but information utilized by the first and second virtual assistants is updated enabling these to operate ever for efficiently. The second virtual assistant can also monitor the correspondence between the radiology technician and the radiologist in the same way as discussed above, where during that correspondence the radiologist speech can also be monitored and what they are looking at - for example they may also pull up a copy of the imaging request during the call, and/or copies of similar imaging requests, and the radiologists gaze can also be tracked to assess what they consider to be important. This information can then be used to support the radiologist and update information on the physician, radiology technician, and even physician.

This process of monitoring the radiology technician and the radiologist can also take place when they are not corresponding with each other, but when they are working on their associated tasks, where the virtual assistants enter dialogue with the relevant person, for example asking questions about performance of the relevant task, and monitoring of how the relevant person responds via speech monitoring and/or gaze monitoring the relevant assistant can support the relevant person, and also update the information on each of the physician, radiology technician and the radiologist.

In an example, the first virtual assistant is configured to utilize one or more of the following to support the technician to carry out the imaging exam of the patient according to the imaging request: information from the medical database, information on the physician, information on previous imaging requests, information on previous exams carried out on the basis of previous imaging requests, information on previous reports generated from patient imagery generated from previous exams carried out on the basis of previous imaging requests.

In an example, the first virtual assistant is configured to utilize the following to support the technician to carry out the imaging exam of the patient according to the imaging request: the technician's response to dialogue with the second virtual assistant, wherein the dialogue with the second virtual assistant comprises utilization of a VDU and/or a chatbot implemented by the second virtual assistant.

In an example, the first virtual assistant is configured to receive input from an eye tracking apparatus and/or from a microphone and/or camera, and the technician's response to dialogue with the first virtual assistant comprises assessment of the technician's gaze direction with respect to content presented on the VDU and/or assessment of the technician's verbal response to content presented via the chatbot and/or assessment of the technician's facial expression response to the dialogue.

In an example, the first virtual assistant is configured to receive written and/or verbal correspondence between the technician and the physician and the first virtual assistant is configured to utilize the written and/or verbal correspondence between the technician and the physician to support the technician to carry out the imaging exam of the patient according to the imaging request. Additionally or alternatively the first virtual assistant is configured to receive written and/or verbal correspondence between the technician and the medical expert and the first virtual assistant is configured to utilize the written and/or verbal correspondence between the technician and the medical expert to support the technician to carry out the imaging exam of the patient according to the imaging request.

In an example, the second virtual assistant is configured to receive input from an eye tracking apparatus and/or from a microphone and/or camera, and the medical expert's response to dialogue with the second virtual assistant comprises assessment of the medical expert's gaze direction with respect to content presented on the VDU and/or assessment of the medical expert's verbal response to content presented via the chatbot and/or assessment of the medical expert's facial expression response to the dialogue.

In an example, the second virtual assistant is configured to utilize one or more of the following to support the medical expert to review the patient imagery and generate the report for the physician: information on the physician, information on the technician, information from the medical database.

In an example, the second virtual assistant is configured to receive written and/or verbal correspondence between the medical expert and the physician and the second virtual assistant is configured to utilize the written and/or verbal correspondence between the medical expert and the physician to support the medical expert to review the patient imagery and generate a report for the physician. Additionally or alternatively the second virtual assistant is configured to receive written and/or verbal correspondence between the medical expert and the technician and the second virtual assistant is configured to utilize the written and/or verbal correspondence between the medical expert and the technician to support the medical expert to review the patient imagery and generate a report for the physician.

In an example, based on the support provided to the technician to carry out the imaging exam of the patient according to the imaging request the first virtual assistant is configured to update the information on the physician and/or to update the information on the technician and/or to update the information on the medical expert. Additionally or alternatively based on the support provided to the medical expert to review the patient imagery and generate the report for the physician the second virtual assistant is configured to update the information on the physician and/or to update the information on the technician and/or to update the information on the medical expert.

Fig. 6 shows a medical imaging processing method 100, comprising:
supporting 110, by a first virtual assistant, a physician to prepare an imaging request for a patient, wherein the imaging request is to be used by a technician who will carry out an imaging exam of the patient to generate patient imagery, and wherein the first virtual assistant utilizes one or more of the following to support the physician to prepare the imaging request: information on the patient, information from a medical database, information on the physician, information on previous imaging requests, the physician's response to dialogue with the first virtual assistant, wherein the dialogue with the first virtual assistant comprises utilization of a VDU and/or a chatbot implemented by the first virtual assistant;
supporting 120, by a second virtual assistant, the technician to carry out the imaging exam of the patient according to the imaging request to generate the patient imagery, and wherein the second virtual assistant utilizes one or more of the following to support the technician to carry out the imaging exam of the patient according to the imaging request: information on the patient, information on the technician, information on a medical expert who will review the patient imagery and generate a report for the physician; and
supporting 130, by a third virtual assistant, the medical expert to review the patient imagery and generate the report for the physician, and wherein the third virtual assistant utilizes one or more of the following to support the medical expert to review the patient imagery and generate the report for the physician: information on the patient, information on the medical expert, information on previous reports generated from patient imagery, the medical expert's response to dialogue with the third virtual assistant, wherein the dialogue with the third virtual assistant comprises utilization of a VDU and/or a chatbot implemented by the third virtual assistant.

In an example, the first virtual assistant utilizes the following to support the physician to prepare the imaging request: information on the technician who will carry out the imaging exam of the patient according to the imaging request to generate the patient imagery.

In an example, the first virtual assistant utilizes the following to support the physician to prepare the imaging request: information on previous exams carried out on the basis of previous imaging requests,

In an example, the first virtual assistant utilizes the following to support the physician to prepare the imaging request: information on the medical expert who will review the patient imagery and generate the report for the physician.

In an example, the first virtual assistant utilizes the following to support the physician to prepare the imaging request: information on previous reports generated from patient imagery acquired in accordance with previous imaging requests.

In an example, the method comprises receiving by the first virtual assistant input from an eye tracking apparatus and/or from a microphone and/or from a camera, and wherein the physician's response to dialogue with the first virtual assistant comprises assessment of the physician's gaze direction with respect to content presented on the VDU and/or assessment of the physician's verbal response to content presented via the chatbot and/or assessment of the physician's facial expression response to the dialogue.

In an example, the method comprises receiving by the first virtual assistant written and/or verbal correspondence between the physician and the technician and utilizing by the first virtual assistant the written and/or verbal correspondence between the physician and the technician to support the physician to prepare the imaging request.

In an example, the method comprises receiving by the first virtual assistant written and/or verbal correspondence between the physician and the medical expert and utilizing by the first virtual assistant the written and/or verbal correspondence between the physician and the medical expert to support the physician to prepare the imaging request.

In an example, the second virtual assistant utilizes one or more of the following to support the technician to carry out the imaging exam of the patient according to the imaging request: information from the medical database, information on the physician, information on previous imaging requests, information on previous exams carried on the basis of previous imaging requests, information on previous reports generated from patient imagery generated from previous exams carried out on the basis of previous imaging requests.

In an example, the second virtual assistant utilizes the following to support the technician to carry out the imaging exam of the patient according to the imaging request: the technician's response to dialogue with the second virtual assistant. The dialogue with the second virtual assistant comprises utilization of a VDU and/or a chatbot implemented by the second virtual assistant.

In an example, the method comprises receiving by second virtual assistant input from an eye tracking apparatus and/or from a microphone and/or from a camera, and the technician's response to dialogue with the second virtual assistant comprises assessing the technician's gaze direction with respect to content presented on the VDU and/or assessing the technician's verbal response to content presented via the chatbot and/or assessing the technician's facial expression response to the dialogue.

In an example, the method comprises receiving by the second virtual assistant written and/or verbal correspondence between the technician and the physician and utilizing by the second virtual assistant the written and/or verbal correspondence between the technician and the physician to support the technician to carry out the imaging exam of the patient according to the imaging request.

In an example, the method comprises receiving by the second virtual assistant written and/or verbal correspondence between the technician and the medical expert and utilizing by the second virtual assistant the written and/or verbal correspondence between the technician and the medical expert to support the technician to carry out the imaging exam of the patient according to the imaging request.

In an example, the method comprises receiving by the third virtual assistant input from an eye tracking apparatus and/or from a microphone and/or from a camera, and the medical expert's response to dialogue with the third virtual assistant comprises assessing the medical expert's gaze direction with respect to content presented on the VDU and/or assessing the medical expert's verbal response to content presented via the chatbot and/or assessing of the medical expert's facial expression response to the dialogue.

In an example, the third virtual assistant utilizes one or more of the following to support the medical expert to review the patient imagery and generate the report for the physician: information on the physician, information on the technician, information from the medical database.

In an example, the method comprises receiving by the third virtual assistant written and/or verbal correspondence between the medical expert and the physician and utilizing by the third virtual assistant the written and/or verbal correspondence between the medical expert and the physician to support the medical expert to review the patient imagery and generate a report for the physician.

In an example, the method comprises receiving by the third virtual assistant written and/or verbal correspondence between the medical expert and the technician and utilizing by the third virtual assistant the written and/or verbal correspondence between the medical expert and the technician to support the medical expert to review the patient imagery and generate a report for the physician.

In an example, based on the support provided to the physician to prepare the imaging request the method comprises updating by the first virtual assistant the information on the physician and/or updating by the first virtual assistant the information on the technician and/or updating by the first virtual assistant the information on the medical expert.

In an example, based on the support provided to the technician to carry out the imaging exam of the patient according to the imaging request the method comprises updating by the second virtual assistant the information on the physician and/or updating by the second virtual assistant the information on the technician and/or updating by the second virtual assistant the information on the medical expert.

In an example, based on the support provided to the medical expert to review the patient imagery and generate the report for the physician the method comprises updating by the third virtual assistant the information on the physician and/or updating by the third virtual assistant the information on the technician and/or updating by the third virtual assistant the information on the medical expert.

The method of Fig. 6 can however operate without one of the three parties receiving virtual assistant support. Such a medical imaging processing method 100 comprises:
supporting 110, by a first virtual assistant, a physician to prepare an imaging request for a patient, wherein the imaging request is to be used by a technician who will carry out an imaging exam of the patient to generate patient imagery, and wherein the first virtual assistant utilizes one or more of the following to support the physician to prepare the imaging request: information on the patient, information from a medical database, information on the physician, information on previous imaging requests, the physician's response to dialogue with the first virtual assistant, wherein the dialogue with the first virtual assistant comprises utilization of a VDU and/or a chatbot implemented by the first virtual assistant;
supporting 120, by a second virtual assistant, the technician to carry out the imaging exam of the patient according to the imaging request to generate the patient imagery, and wherein the second virtual assistant utilizes one or more of the following to support the technician to carry out the imaging exam of the patient according to the imaging request: information on the patient, information on the technician, information on a medical expert who will review the patient imagery and generate a report for the physician; and
wherein the patient imagery is provided to the medical expert to review and generate the report for the physician.

In an example, the first virtual assistant utilizes the following to support the physician to prepare the imaging request: information on the technician who will carry out the imaging exam of the patient according to the imaging request to generate the patient imagery.

In an example, the first virtual assistant utilizes the following to support the physician to prepare the imaging request: information on previous exams carried out on the basis of previous imaging requests.

In an example, the first virtual assistant utilizes the following to support the physician to prepare the imaging request: information on the medical expert who will review the patient imagery and generate the report for the physician.

In an example, the first virtual assistant utilizes the following to support the physician to prepare the imaging request: information on previous reports generated from patient imagery acquired in accordance with previous imaging requests.

In an example, the method comprises receiving by the first virtual assistant input from an eye tracking apparatus and/or from a microphone and/or camera, and the physician's response to dialogue with the first virtual assistant comprises assessment of the physician's gaze direction with respect to content presented on the VDU and/or assessment of the physician's verbal response to content presented via the chatbot and/or assessment of the physician's facial expression response to the dialogue.

In an example, the method comprises receiving by the first virtual assistant written and/or verbal correspondence between the physician and the technician and utilizing by the first virtual assistant the written and/or verbal correspondence between the physician and the technician to support the physician to prepare the imaging request.

In an example, the method comprises receiving by the first virtual assistant written and/or verbal correspondence between the physician and the medical expert and utilizing by the first virtual assistant the written and/or verbal correspondence between the physician and the medical expert to support the physician to prepare the imaging request.

In an example, the second virtual assistant utilizes one or more of the following to support the technician to carry out the imaging exam of the patient according to the imaging request: information from the medical database, information on the physician, information on previous imaging requests, information on previous exams carried on the basis of previous imaging requests, information on previous reports generated from patient imagery generated from previous exams carried out on the basis of previous imaging requests.

In an example, the second virtual assistant utilizes the following to support the technician to carry out the imaging exam of the patient according to the imaging request: the technician's response to dialogue with the second virtual assistant, wherein the dialogue with the second virtual assistant comprises utilization of a VDU and/or a chatbot implemented by the second virtual assistant.

In an example, the method comprises receiving by the second virtual assistant input from an eye tracking apparatus and/or from a microphone and/or camera, and the technician's response to dialogue with the second virtual assistant comprises assessing the technician's gaze direction with respect to content presented on the VDU and/or assessing the technician's verbal response to content presented via the chatbot and/or assessing of the technician's facial expression response to the dialogue.

In an example, the method comprises receiving by the second virtual assistant written and/or verbal correspondence between the technician and the physician and utilizing by the second virtual assistant the written and/or verbal correspondence between the technician and the physician to support the technician to carry out the imaging exam of the patient according to the imaging request.

In an example, based on the support provided to the physician to prepare the imaging request the method comprises updating by the first virtual assistant the information on the physician and/or updating by the first virtual assistant the information on the technician and/or updating by the first virtual assistant the information on the medical expert.

In an example, based on the support provided to the technician to carry out the imaging exam of the patient according to the imaging request the method comprises updating by the second virtual assistant the information on the physician and/or updating by the second virtual assistant the information on the technician and/or updating by the second virtual assistant the information on the medical expert.

As detailed above the method of Fig. 6 can operate without one of the three parties receiving virtual assistant support. Another such a medical imaging processing method 100 comprises:
supporting 110, by a first virtual assistant, a physician to prepare an imaging request for a patient, wherein the imaging request is to be used by a technician who will carry out an imaging exam of the patient to generate patient imagery, and wherein the first virtual assistant utilizes one or more of the following to support the physician to prepare the imaging request: information on the patient, information from a medical database, information on the physician, information on previous imaging requests, the physician's response to dialogue with the first virtual assistant, wherein the dialogue with the first virtual assistant comprises utilization of a VDU and/or a chatbot implemented by the first virtual assistant; providing the imaging request to the technician to carry out the imaging exam of the patient according to the imaging request to generate the patient imagery; and
supporting 130, by a second virtual assistant, the medical expert to review the patient imagery and generate the report for the physician, and wherein the second virtual assistant utilizes one or more of the following to support the medical expert to review the patient imagery and generate the report for the physician: information on the patient, information on the medical expert, information on previous reports generated from patient imagery, the medical expert's response to dialogue with the second virtual assistant, wherein the dialogue with the second virtual assistant comprises utilization of a VDU and/or a chatbot implemented by the second virtual assistant.

In an example, the first virtual assistant utilizes the following to support the physician to prepare the imaging request: information on the technician who will carry out the imaging exam of the patient according to the imaging request to generate the patient imagery.

In an example, the first virtual assistant utilizes the following to support the physician to prepare the imaging request: information on previous exams carried out on the basis of previous imaging requests.

In an example, the first virtual assistant utilizes the following to support the physician to prepare the imaging request: information on the medical expert who will review the patient imagery and generate the report for the physician.

In an example, the first virtual assistant utilizes the following to support the physician to prepare the imaging request: information on previous reports generated from patient imagery acquired in accordance with previous imaging requests.

In an example, the method comprises receiving by the first virtual assistant input from an eye tracking apparatus and/or from a microphone and/or camera, and wherein the physician's response to dialogue with the first virtual assistant comprises assessing the physician's gaze direction with respect to content presented on the VDU and/or assessing the physician's verbal response to content presented via the chatbot and/or assessing the physician's facial expression response to the dialogue.

In an example, the method comprises receiving by the first virtual assistant written and/or verbal correspondence between the physician and the technician and utilizing by the first virtual assistant the written and/or verbal correspondence between the physician and the technician to support the physician to prepare the imaging request.

In an example, the method comprises receiving by the first virtual assistant written and/or verbal correspondence between the physician and the medical expert and utilizing by the first virtual assistant the written and/or verbal correspondence between the physician and the medical expert to support the physician to prepare the imaging request.

In an example, the method comprises receiving by the second virtual assistant input from an eye tracking apparatus and/or from a microphone and/or camera, and the medical expert's response to dialogue with the second virtual assistant comprises assessing the medical expert's gaze direction with respect to content presented on the VDU and/or assessing the medical expert's verbal response to content presented via the chatbot and/or assessing the medical expert's facial expression response to the dialogue.

In an example, the second virtual assistant utilizes one or more of the following to support the medical expert to review the patient imagery and generate the report for the physician: information on the physician, information on the technician, information from the medical database.

In an example, the method comprises receiving by the second virtual assistant written and/or verbal correspondence between the medical expert and the physician and utilizing by the second virtual assistant the written and/or verbal correspondence between the medical expert and the physician to support the medical expert to review the patient imagery and generate a report for the physician.

In an example, the method comprises receiving by the second virtual assistant written and/or verbal correspondence between the medical expert and the technician and utilizing by the second virtual assistant the written and/or verbal correspondence between the medical expert and the technician to support the medical expert to review the patient imagery and generate a report for the physician.

In an example, based on the support provided to the physician to prepare the imaging request the method comprises updating by the first virtual assistant the information on the physician and/or updating by the first virtual assistant the information on the technician and/or updating by the first virtual assistant the information on the medical expert.

In an example, based on the support provided to the medical expert to review the patient imagery and generate the report for the physician the method comprises updating by the second virtual assistant the information on the physician and/or updating by the third virtual assistant the information on the technician and/or updating by the third virtual assistant the information on the medical expert.

As detailed above the method of Fig. 6 can operate without one of the three parties receiving virtual assistant support. Another such a medical imaging processing method 100 comprises:
supporting 120, by a first virtual assistant, a technician to carry out an imaging exam of a patient according to an imaging request prepared by a physician to generate patient imagery, and wherein the first virtual assistant utilizes one or more of the following to support the technician to carry out the imaging exam of the patient according to the imaging request: information on the patient, information on the technician, information on a medical expert who will review the patient imagery and generate a report for the physician; and
supporting 130, by a second virtual assistant, the medical expert to review the patient imagery and generate the report for the physician, and wherein the second virtual assistant utilizes one or more of the following to support the medical expert to review the patient imagery and generate the report for the physician: information on the patient, information on the medical expert, information on previous reports generated from patient imagery, the medical expert's response to dialogue with the third virtual assistant, wherein the dialogue with the third virtual assistant comprises utilization of a VDU and/or a chatbot implemented by the third virtual assistant.

In an example, the first virtual assistant utilizes one or more of the following to support the technician to carry out the imaging exam of the patient according to the imaging request: information from the medical database, information on the physician, information on previous imaging requests, information on previous exams carried on the basis of previous imaging requests, information on previous reports generated from patient imagery generated from previous exams carried out on the basis of previous imaging requests.

In an example, the first virtual assistant utilizes the following to support the technician to carry out the imaging exam of the patient according to the imaging request: the technician's response to dialogue with the first virtual assistant. The dialogue with the first virtual assistant comprises utilizing a VDU and/or a chatbot implemented by the first virtual assistant.

In an example, the method comprises receiving by the first virtual assistant input from an eye tracking apparatus and/or from a microphone and/or camera, and wherein the technician's response to dialogue with the first virtual assistant comprises assessing the technician's gaze direction with respect to content presented on the VDU and/or assessing the technician's verbal response to content presented via the chatbot and/or assessing the technician's facial expression response to the dialogue.

In an example, the method comprises receiving by the first virtual assistant written and/or verbal correspondence between the technician and the physician and utilizing by the first virtual assistant the written and/or verbal correspondence between the technician and the physician to support the technician to carry out the imaging exam of the patient according to the imaging request.

In an example, the method comprises receiving by the first virtual assistant written and/or verbal correspondence between the technician and the medical expert and utilizing by the first virtual assistant the written and/or verbal correspondence between the technician and the medical expert to support the technician to carry out the imaging exam of the patient according to the imaging request.

In an example, the method comprises receiving by the second virtual assistant input from an eye tracking apparatus and/or from a microphone and/or camera, and the medical expert's response to dialogue with the second virtual assistant comprises assessing the medical expert's gaze direction with respect to content presented on the VDU and/or assessing the medical expert's verbal response to content presented via the chatbot and/or assessing the medical expert's facial expression response to the dialogue.

In an example, the second virtual assistant utilizes one or more of the following to support the medical expert to review the patient imagery and generate the report for the physician: information on the physician, information on the technician, information from the medical database.

In an example, the method comprises receiving by the second virtual assistant written and/or verbal correspondence between the medical expert and the physician and utilizing by the second virtual assistant the written and/or verbal correspondence between the medical expert and the physician to support the medical expert to review the patient imagery and generate a report for the physician.

In an example, the method comprises receiving by the second virtual assistant written and/or verbal correspondence between the medical expert and the technician and utilizing by the second virtual assistant the written and/or verbal correspondence between the medical expert and the technician to support the medical expert to review the patient imagery and generate a report for the physician.

In an example, based on the support provided to the technician to carry out the imaging exam of the patient according to the imaging request the method comprises updating by the first virtual assistant the information on the physician and/or updating by the first virtual assistant the information on the technician and/or updating by the first virtual assistant the information on the medical expert.

In an example, based on the support provided to the medical expert to review the patient imagery and generate the report for the physician the method comprises updating by the second virtual assistant the information on the physician and/or updating by the second virtual assistant the information on the technician and/or updating by the second virtual assistant the information on the medical expert.

Thus new systems and methods have been developed to improve the efficiency and efficacy of imaging request and report creation in a radiology workflow. The systems and or methods in a detailed embodiment is a self-learning network with two/three collaborating voice / chatbot-enabled virtual assistants (or indeed only one virtual assistant utilized by all users) in a radiology workflow, in which:
1. A first virtual assistant supports a referring physician in creating an imaging request
2. A second virtual assistant supports a radiology department user in executing the request (the second virtual assistant can be the first virtual assistant)
3. At least one virtual assistant measures the user responses during the task, in relation to the dialogue performed by this virtual assistant with the user
4. At least one virtual assistant adapts (personalizes) the dialogue to the measured user response (over time) and the known experience level of this user
5. All virtual assistants collaboratively work towards optimal imaging requests and imaging reports to reach a common goal: improving measured imaging effectiveness and efficiency in a radiology workflow

Thus, systems and methods for electronic requests for diagnostic imaging is provided. This allows more efficient and effective imaging request formulation, execution, reading, reporting and review by all personnel involved in the radiology workflow.

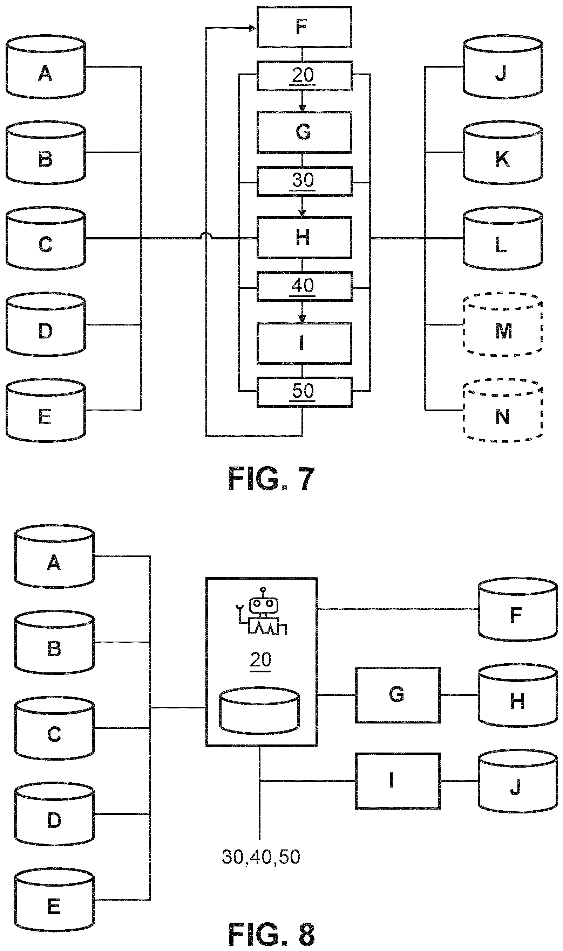

The medical imaging processing systems and medical imaging processing methods are now described in specific detail with respect to detailed embodiments, where reference is made to Figs. 7-13.

It is to be noted that a specific detailed embodiment of the system has a virtual assistant supporting a physician, another virtual assistant (or the same virtual assistant) supporting a radiology technician, and another virtual assistant (or the same virtual assistant) supporting a radiologist. However, in another system no virtual assistant supports the radiologist. Also, in another system no virtual assistant supports the physician. Additionally, in another system no virtual assistant supports the radiology technician.

The following describes the situation, where all three of the physician, the radiology technician and the radiologist are supported by virtual assistant(s), which does not limit what the different systems enable. This is because not all three of the physician, the radiology technician and the radiologist need to have virtual assistant support. All three can have support, but only the physician and the radiology technician can have virtual assistant support, only the physician and the radiologist can have virtual assistant support, and only the radiologist and the radiology technician can have virtual assistant support.

As detailed above, the specific detailed embodiment now described relates to the situation where all of the physician, the radiology technician and the radiologist are supported by virtual assistant(s), where there can be one virtual assistant supporting all users, one virtual assistant supporting two users and a second supporting the third user, or three virtual assistants, each supporting a different user.

The system and or method developed is a self-learning network with at least two collaborating voice / chatbot-enabled virtual assistants in a radiology workflow, in which:
A first virtual assistant supports a referring physician in creating an imaging request
A second virtual assistant supports a radiology department user in executing the request
At least one virtual assistant measures the user responses during the task, in relation to the dialogue performed by this virtual assistant with the user
At least one virtual assistant adapts (personalizes) the dialogue to the measured user response (over time) and the known experience level of this user
All virtual assistants collaboratively work towards optimal imaging requests and imaging reports to reach a common goal: improving measured imaging effectiveness and efficiency in a radiology workflow

Fig. 7 shows a system diagram overview of the new development, which is used to discuss the details of how the system and method can be built and used. The (various instances of the) Imaging Virtual Assistant is linked to all stages/users. The system collects data from each stage and allows the system to improve and prompt the user at each stage of the process. In Fig. 7 "electronic (patient) health records" is signified by A, "imaging request protocols" is signified by B, "medical knowledge" is signified by C, "imaging requests with meta data" is signified by D, "imaging reports with metadata" is signified by E, "referring physician (write imaging request and review imaging report)" is signified by F, "first imaging virtual assistant 1" is signified by 20, "second imaging virtual assistant 2" is signified by 30, "third imaging virtual assistant 3" is signified by 40, "fourth imaging virtual assistant 4" is signified by 50 (it is to be noted that virtual assistants 30 and 40 can be implemented as a single virtual assistant, and it is to be noted that a single virtual assistant formed from 30 and 40 can be implemented as one whole single virtual assistant with virtual assistant 50). Continuing with Fig. 7 "diagnostic radiologists/radiology lab (scan type and settings)" is signified by G, "radiologic technologist (perform scan and send images)" is signified by H, "diagnostic radiologists (read images and write report)" is signified by I, "staff experience, specialisation and preference" is signified by J, "staff response measurements" is signified by K, "workflow measurements" is signified by L, "imaging centre database" is signified by M, and "equipment and staff schedules" is signified by N.

Fig. 8 shows a sub-system diagram of an embodiment of an Imaging Request Virtual Assistant in the system. In this section it is explained how such an assistant can aid a referring physician in making an effective imaging request. Thus, this virtual assistant can be virtual assistant 20 as detailed in Fig. 7. In Fig. 8 "electronic (patient) health records" is signified by A, "imaging request protocols" is signified by B, "medical knowledge" is signified by C, "imaging requests with metadata" is signified by D, "imaging reports with metadata" is signified by E, "imaging virtual assistant" is signified by 20, "other imaging Virtual assistants" is signified by 30, 40, 50, "staff experience, specialisation and preference" is signified by F, "emotion/interaction/speech/gaze pattern analysis" is signified by G, "staff response measurements" is signified by H, "clarification calls, re-exams, duration" is signified by I, "workflow measurements" is signified by J.

### Imaging Virtual Assistant for the Referring Physician

Instead of a form-filling user interface, a voice-enabled or chatbot-like virtual assistant has been developed that supports the referring physician in creating an imaging request. The Imaging Virtual Assistant collects all available prior knowledge of a patient and asks a minimal set of key questions in the most efficient order to get the imaging request formulated. This virtual assistant could already be present during the initial patient consultation to capture information for the imaging request creation (see for example: https://deeptalk-ai.com). The referring physician then only has to review a smartly summarized request overview and submit if satisfied. This makes the imaging request process mush faster and intuitive for the referring physician while at the same time assuring quality of the imaging requests, to avoid errors and recalls by the radiology department as described earlier. Based on various databases, as discussed below, the assistant generates the most efficient dialogue to get the required information from the referring physician to create the desired imaging request. This type of dialogue generation management is constantly tailored to:
The level of experience, specialisation, and preferences of the referring physician
How the referring physician responds to the dialogue of the virtual assistant, in terms of response times, eye gaze patterns, language use and/or measured emotions
Knowledge about prior imaging requests and imaging reports from/to the referring physician, and possibly the targeted radiology staff, which resulted in workflow (in-)efficiency

### Databases

As illustrated in Fig. 8, the Imaging Request Virtual Assistant is connected to several databases and use this data to auto complete the form.

Electronic Patient Health Records Database - The Imaging Request Virtual Assistant will pull in the patent electronic records and use this data to auto complete all the patient related parts of the imaging request. It may also propose certain exam types based on recent medical history Imaging request writing standards and protocols database - The Imaging Request Virtual Assistant will pull in the current standards and protocols for writing a complete, clear and protocol adherent imaging request. This database may also include the related exam cards and system settings for specific exams, which can then be included in the request.

A medical knowledge database may support medical reasoning with information from the previous two databases, and may contain heuristics of possibly underlying conditions in relation to symptoms, and relate these to certain preferred imaging exam types

Imaging request database with metadata - The Imaging Request Virtual Assistant is self-learning and builds up its own knowledge of effective imaging requests that typically result in high workflow efficiency and effectiveness. Besides adherence to the standards and protocols, these imaging requests may contain extra metadata (comments, clarifications or attention areas) that improve processing of the imaging request by the various users in the targeted radiology department. The extra metadata may depend on the experience, specialization, or preference of the targeted radiology staff, and may be added automatically by the virtual assistant in the currently created imaging request

Imaging reports database with metadata - Similar to the imaging request database, this database contains imaging reports with linked metadata on what made the report ineffective or effective in relation to the related imaging request and the radiology staff processing the request.

Staff experience, specialization, and preference database - The Imaging Request Virtual Assistant uses this information to (1) tailor the dialogue to the referring physician's experience, specialization and preference, and (2) make sure that the imaging requests meets the experience, specialization and preferences of the targeted radiology staff. This database may be updated with information from the staff response measurement database. For example, if a radiology technician typically responds with hesitations to certain imaging requests, this may be stored as a low experience/specialization item in the experience, specialization, and preference database.

Staff response measurements - the dialogue and interaction with the Imaging Request Virtual Assistant and other systems used is constantly monitored. Log files, speech analysis, gaze pattern analysis, facial expression monitoring and/or for example emotion sensing are used to (1) tailor the dialogue of the Imaging Request Virtual Assistant of the referring physician, (2) update Staff experience, specialization, and preference database, and (3) provide input to workflow measurements database. E.g., the system can collect statistics and measure how much time is spent on each section of the image request.

Workflow measurements database - stores timings and (measured) events that impact workflow efficiency and effectiveness, such as duration of the request formulation, exam taking, reading, and reporting, and undesired events such as clarification calls and re-exams. This information is used by the Imaging Request Virtual Assistant as reference data for its self-learning to create optimal imaging request. This will save time for the image request reading the next time, and also provide useful statistics to management.

This information above can be considered to be: information on the patient, information from a medical database, information on the physician, information on previous imaging requests, the physician's response to dialogue with first virtual assistant 20, information on the technician, information on a medical expert who will review the patient imagery and generate a report for the physician, information on previous reports generated from patient imagery, the medical expert's response to dialogue with the virtual assistant 50, information on previous exams carried out on the basis of previous imaging requests, information on previous exams carried out on the basis of previous imaging requests, information on previous reports generated from patient imagery generated from previous exams carried out on the basis of previous imaging requests, the technician's response to dialogue with the virtual assistant/s) 30/40

### Connected Imaging Virtual Assistant for the Radiology Staff

As shown in Fig. 7, various connected virtual assistants, or various instances of a single virtual assistant, are used that support(s) multiple roles in the radiology workflow: e.g., the patient, exam scheduler, radiology technician, and radiologist.

### Radiology Technician (Perform Imaging)

The technician is the person working with the patient and the imaging system to perform the imaging. The Radiologic Technologist reads the request and if he has a clear description of what is asked, executes the scan with the patient. His (instance of) the imaging request virtual assistant may start a dialogue on what is required for this exam in terms of, e.g., coil placement, positions, patient guidance, and so on, and automatically set certain equipment settings. As the Imaging Request Virtual Assistant has access to the patient electronic medical records he may also use this to warn the technician if special steps need to be taken, for example a patient with pacemaker going for an MRI. The Imaging Request Virtual Assistant also knows the preferences of the radiologists who will be reading the images and may accordingly adjust certain imaging settings or add certain extra information.

If there are any measured hesitations by the technician, the virtual assistant may adapt its guidance by providing extra information to the technician. The virtual assistant may also do analysis of the root cause of the unclarity: if (1) it is related to unclear / missing data in the imaging request, or (2) related to inexperience of the technician, and accordingly (1) update the metadata in the imaging requests database or (2) the staff experience, specialisation, and preference database. It is to be noted that different databases are described here, but there could be just a single database, and also in the disclosure reference to "database" then does not necessarily mean a single database bit can mean a plurality of databases. This way the next time the Imaging Request Virtual Assistant assists the referring physician in creating an imaging request, it will know what this centre/user likes to have in its request and or guidance.

### Diagnostic Radiologists (Read images and write report)

Similar to the support for the referring physician, (an instance of) the Imaging Request Virtual Assistant for the radiologist can support the report writing. The system may know 'standard reports' and will suggest these to the radiologist and prompt him to leverage these to speed up and harmonize report writing, while tailoring it with possible extra information to meet the experience, specialization, and preferences of the referring physician (as stored in the related database).

Similar to the referring physician stage, the software may monitor the person reading the images. The system may measure how much time is spent on each section of the images, analyse gaze patterns, analyse speech patterns, analyse facial expressions and sense the radiologist's emotions. Overly long or short attention to certain image segments, doubt, frustration or any other indications of issues with either the imaging request or the images taken by the technician, may be related back to its root cause for the network of virtual assistants to learn from (see the description under technician).

If the radiologists is unclear and makes a phone call to discussion the analysis, the system learns from this and next time knows what this user likes to know to interpret and understand the results. If the user looks up information, again the system will know what information this user needs, stores this in the Staff experience, specialization and preference database. The referring physician's assistant can then adapt and suggest this the next time during image request writing.

The system employs AI and creates a diagnosis and report efficiency index. This will track current efficiency and monitor inefficiencies. From this the system gets better at predicting what information is missing from the data and prompts them for this information the next time they read the results and creates the report. This will save time for the image request reading the next time.

### Closing the loop: back to the referring physician (Read report and review images)

This is the final step in the image request journey. The referring physician who ordered the imaging now gets the completed report and imaging. In line with the previous steps, if any clarification communication is made between the referring physician and the diagnostic radiologist, the Imaging Request Virtual Assistant learns from this and updates its databases, so this does not happen again. This understanding of what constitutes a clear and complete report can be used to provide real-time feedback to the physician writing the report. Again, staff response measurement can be used to track understanding of the report. For example, gaze tracking can be used to assess the Referring Physician cognition, by monitoring the time spent reading the report and viewing the imaging. After root cause analysis this insight may then be used to update (1) the Staff Experience, Specialization, and Preference data for this physician, (2) the metadata linked to the Imaging Request and/or Imaging Report, and/or (3) the Staff Experience, Specialization, and Preference data for the involved radiologist. The root cause analysis itself may also be guided by the stored experience / specialization levels: if the referring physician is highly specialized in the exam at hand, while the technician and / or radiologist is less experienced / specialized, the root cause of the issues may be more likely attributed to the inexperience of the technician or radiologist, and vice versa (unless the root cause is a clear miss in the imaging request, or in the imaging report).

### ADDITIONAL EMBODIMENTS

### Radiology Lab selection and scheduling

Imaging Center database - The Imaging Imaging Virtual Assistant is self-learning and builds up its own Imaging Center preference database. For example, the imaging facility/center may have its own preferences of key information it needs to perform a scan. While facility/centre B may have different preference. This imaging center database will help prompt and imaging requestor to have the key information needed for the intended imaging center. In addition, the Imaging Request Virtual Assistant can make useful suggestions to the referring physician. For example, if the referring Physician is requesting a PET scan, the Imaging Request Virtual Assistant can recommend centres based on positive feedback/experience in the past.

Equipment and staff schedules database - The Imaging Virtual Assistant could also be installed at the Radiology center and have access to equipment and personnel scheduling information. From this the system can auto recommend scheduling, matching equipment, radiologic technologist, diagnostic radiologist to the patient. If the referring physician also request a specific diagnostic radiologist, the Image Request Virtual assistant can also link and schedule those people to the imaging.

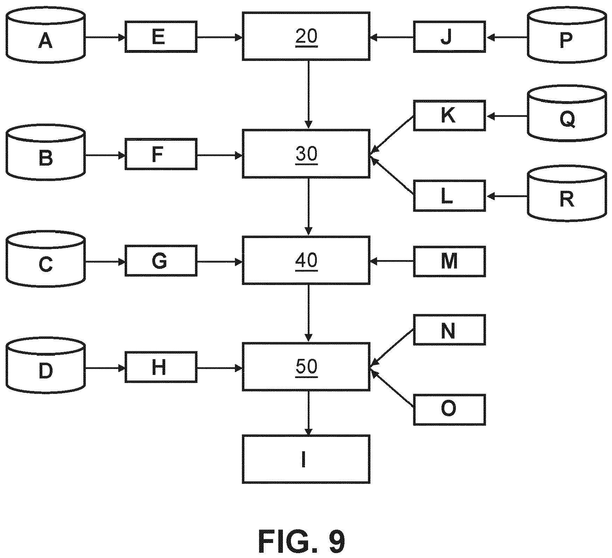

Fig. 9 shows various additional embodiments of automated functionalities that the Imaging Request Virtual Assistant ca provide, most of which have been discussed above. In Fig. 9 "database" is signified by A, "equipment and staff schedules" is signified by B, "electronic patient records" is signified by C, "database of reports" is signified by D, "auto fill fields (AI)" is signified by E, "auto suggest schedule (match lab equipment to patient)" is signified by F, "auto warning system (pacemaker for example)" is signified by G, "auto report writing. Radiologist review suggested reports" is signified by H, "image virtual assistant and referring physician" is signified by 20, "image virtual assistant and radiology lab/centre" is signified by 30, image virtual assistant and radiologic technologist" is signified by 40, "image virtual assistant and diagnostic radiologists" is signified by 50, "referring physician (read report and review images)" is signified by I, "auto suggest radiology centre" is signified by J, "auto suggest radiologic technologist" is signified by K, "auto suggest diagnostic radiologist" is signified by L, "auto suggest optimal radiology settings" is signified by M, "auto image reading (AI) radiologist review suggested diagnosis" is signified by N, "auto collect radiologist statistics (imagery time, report time, clarification calls)" is signified by O, "imaging Centre database" is signified by P, "staff experience, specialisation, preference" is signified by Q, and "staff experience, specialisation, preference" is signified by R.

Thus, in overview, a system and/or method has been developed that uses a self-learning network of collaborating voice / chatbot-enabled virtual assistants, to support one or more tasks:
The referring physician in creating high quality (complete, clear and protocol adherent) imaging requests, in an easy and efficient way
The radiologic technician to correctly perform the exam
The radiologist to correctly read and report on the exam in context of the imaging request The referring physician to receive a correct and well-readable imaging report
The virtual assistants are an effective alternative to form-filling if they are appropriately built and connected to the relevant (patient and medical) data resources. The virtual assistants work interactively, are personalized to the experience level of their users, and act upon the measured response of the users involved in the workflow. This leads to real (radiology) workflow improvement that is enabled by these virtual assistants that adapt to their user's known and measured skill levels, and which work collaboratively to improve the full imaging process.

Eye tracking online movement measurements, are referred to above with further details to be found in the literature, for example in the paper: Using Eye Movements to Evaluate the Cognitive Processes Involved in Text Comprehension by Gary Ranney et al, J Vis Exp. 2014; (83): 50780, and to be found here: https://www.ncbi.nlm.nih.gov/pmc/articles/PMC4089416/

Details on writing a tools party found in literature, for example on: https://www.grammarly.com/

In another exemplary embodiment, a computer program or computer program element is provided that is characterized by being configured to execute the method steps of any of the methods according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A medical imaging processing system (10), comprising:
- a first virtual assistant (20);
- a second virtual assistant (30, 40); and
- a third virtual assistant (50);
wherein the first virtual assistant is configured to support a physician to prepare an imaging request for a patient, wherein the imaging request is to be used by a technician who will carry out an imaging exam of the patient to generate patient imagery, and wherein the first virtual assistant is configured to utilize one or more of the following to support the physician to prepare the imaging request:
information on the patient, information from a medical database, information on the physician, information on previous imaging requests, the physician's response to dialogue with the first virtual assistant, wherein the dialogue with the first virtual assistant comprises utilization of a VDU and/or a chatbot implemented by the first virtual assistant;
wherein the second virtual assistant is configured to support the technician to carry out the imaging exam of the patient according to the imaging request to generate the patient imagery, and wherein the second virtual assistant is configured to utilize one or more of the following to support the technician to carry out the imaging exam of the patient according to the imaging request: information on the patient, information on the technician, information on a medical expert who will review the patient imagery and generate a report for the physician; and
wherein the third virtual assistant is configured to support the medical expert to review the patient imagery and generate the report for the physician, and wherein the third virtual assistant is configured to utilize one or more of the following to support the medical expert to review the patient imagery and generate the report for the physician:
information on the patient, information on the medical expert, information on previous reports generated from patient imagery, the medical expert's response to dialogue with the third virtual assistant, wherein the dialogue with the third virtual assistant comprises utilization of a VDU and/or a chatbot implemented by the third virtual assistant.

2. System according to claim 1, wherein the first virtual assistant is configured to receive input from an eye tracking apparatus and/or from a microphone and/or from a camera, and wherein the physician's response to dialogue with the first virtual assistant comprises assessment of the physician's gaze direction with respect to content presented on the VDU and/or assessment of the physician's verbal response to content presented via the chatbot and/or assessment of the physician's facial expression response to the dialogue.

3. System according to any of claims 1-2, wherein the first virtual assistant is configured to receive written and/or verbal correspondence between the physician and the technician and wherein the first virtual assistant is configured to utilize the written and/or verbal correspondence between the physician and the technician to support the physician to prepare the imaging request and/or wherein the first virtual assistant is configured to receive written and/or verbal correspondence between the physician and the medical expert and wherein the first virtual assistant is configured to utilize the written and/or verbal correspondence between the physician and the medical expert to support the physician to prepare the imaging request.

4. System according to any of claims 1-3, wherein the second virtual assistant is configured to utilize the following to support the technician to carry out the imaging exam of the patient according to the imaging request: the technician's response to dialogue with the second virtual assistant, wherein the dialogue with the second virtual assistant comprises utilization of a VDU and/or a chatbot implemented by the second virtual assistant.

5. System according to any of claims 1-4, wherein the second virtual assistant is configured to receive written and/or verbal correspondence between the technician and the physician and wherein the second virtual assistant is configured to utilize the written and/or verbal correspondence between the technician and the physician to support the technician to carry out the imaging exam of the patient according to the imaging request and/or wherein the second virtual assistant is configured to receive written and/or verbal correspondence between the technician and the medical expert and wherein the second virtual assistant is configured to utilize the written and/or verbal correspondence between the technician and the medical expert to support the technician to carry out the imaging exam of the patient according to the imaging request.

6. System according to any of claims 1-5, wherein the third virtual assistant is configured to receive input from an eye tracking apparatus and/or from a microphone and/or from a camera, and wherein the medical expert's response to dialogue with the third virtual assistant comprises assessment of the medical expert's gaze direction with respect to content presented on the VDU and/or assessment of the medical expert's verbal response to content presented via the chatbot and/or assessment of the medical expert's facial expression response to the dialogue.

7. System according to any of claims 1-6, wherein the third virtual assistant is configured to receive written and/or verbal correspondence between the medical expert and the physician and wherein the third virtual assistant is configured to utilize the written and/or verbal correspondence between the medical expert and the physician to support the medical expert to review the patient imagery and generate a report for the physician and/or wherein the third virtual assistant is configured to receive written and/or verbal correspondence between the medical expert and the technician and wherein the third virtual assistant is configured to utilize the written and/or verbal correspondence between the medical expert and the technician to support the medical expert to review the patient imagery and generate a report for the physician.

8. System according to any of claims 1-7, wherein based on the support provided to the physician to prepare the imaging request the first virtual assistant is configured to update the information on the physician and/or to update the information on the technician and/or to update the information on the medical expert; and/or wherein based on the support provided to the technician to carry out the imaging exam of the patient according to the imaging request the second virtual assistant is configured to update the information on the physician and/or to update the information on the technician and/or to update the information on the medical expert; and/or wherein based on the support provided to the medical expert to review the patient imagery and generate the report for the physician the third virtual assistant is configured to update the information on the physician and/or to update the information on the technician and/or to update the information on the medical expert.

9. A medical imaging processing method (100), comprising:
supporting (110) by a first virtual assistant a physician to prepare an imaging request for a patient, wherein the imaging request is to be used by a technician who will carry out an imaging exam of the patient to generate patient imagery, and wherein the first virtual assistant utilizes one or more of the following to support the physician to prepare the imaging request:
information on the patient, information from a medical database, information on the physician, information on previous imaging requests, the physician's response to dialogue with the first virtual assistant, wherein the dialogue with the first virtual assistant comprises utilization of a VDU and/or a chatbot implemented by the first virtual assistant;
supporting (120) by a second virtual assistant the technician to carry out the imaging exam of the patient according to the imaging request to generate the patient imagery, and wherein the second virtual assistant utilizes one or more of the following to support the technician to carry out the imaging exam of the patient according to the imaging request:
information on the patient, information on the technician, information on a medical expert who will review the patient imagery and generate a report for the physician; and
supporting (130) by a third virtual assistant the medical expert to review the patient imagery and generate the report for the physician, and wherein the third virtual assistant utilizes one or more of the following to support the medical expert to review the patient imagery and generate the report for the physician:
information on the patient, information on the medical expert, information on previous reports generated from patient imagery, the medical expert's response to dialogue with the third virtual assistant, wherein the dialogue with the third virtual assistant comprises utilization of a VDU and/or a chatbot implemented by the third virtual assistant.

10. A medical imaging processing system (10), comprising:
- a first virtual assistant (20); and
- a second virtual assistant (30, 40);
wherein the first virtual assistant is configured to support a physician to prepare an imaging request for a patient, wherein the imaging request is to be used by a technician who will carry out an imaging exam of the patient to generate patient imagery, and wherein the first virtual assistant is configured to utilize one or more of the following to support the physician to prepare the imaging request:
information on the patient, information from a medical database, information on the physician, information on previous imaging requests, the physician's response to dialogue with the first virtual assistant, wherein the dialogue with the first virtual assistant comprises utilization of a VDU and/or a chatbot implemented by the first virtual assistant;
wherein the second virtual assistant is configured to support the technician to carry out the imaging exam of the patient according to the imaging request to generate the patient imagery, and wherein the second virtual assistant is configured to utilize one or more of the following to support the technician to carry out the imaging exam of the patient according to the imaging request:
information on the patient, information on the technician, information on a medical expert who will review the patient imagery and generate a report for the physician; and
wherein the patient imagery is to be provided to the medical expert to review and generate the report for the physician.

11. A medical imaging processing method (100), comprising:
supporting (110) by a first virtual assistant a physician to prepare an imaging request for a patient, wherein the imaging request is to be used by a technician who will carry out an imaging exam of the patient to generate patient imagery, and wherein the first virtual assistant utilizes one or more of the following to support the physician to prepare the imaging request:
information on the patient, information from a medical database, information on the physician, information on previous imaging requests, the physician's response to dialogue with the first virtual assistant, wherein the dialogue with the first virtual assistant comprises utilization of a VDU and/or a chatbot implemented by the first virtual assistant;
supporting (120) by a second virtual assistant the technician to carry out the imaging exam of the patient according to the imaging request to generate the patient imagery, and wherein the second virtual assistant utilizes one or more of the following to support the technician to carry out the imaging exam of the patient according to the imaging request:
information on the patient, information on the technician, information on a medical expert who will review the patient imagery and generate a report for the physician; and
wherein the patient imagery is provided to the medical expert to review and generate the report for the physician.

12. A medical imaging processing system (10), comprising:
- a first virtual assistant (20); and
- a second virtual assistant (50);
wherein the first virtual assistant is configured to support a physician to prepare an imaging request for a patient, wherein the imaging request is to be used by a technician who will carry out an imaging exam of the patient to generate patient imagery, and wherein the first virtual assistant is configured to utilize one or more of the following to support the physician to prepare the imaging request:
information on the patient, information from a medical database, information on the physician, information on previous imaging requests, the physician's response to dialogue with the first virtual assistant, wherein the dialogue with the first virtual assistant comprises utilization of a VDU and/or a chatbot implemented by the first virtual assistant;
wherein the imaging request is to be provided to the technician to carry out the imaging exam of the patient according to the imaging request to generate the patient imagery; and
wherein the second virtual assistant is configured to support the medical expert to review the patient imagery and generate the report for the physician, and wherein the second virtual assistant is configured to utilize one or more of the following to support the medical expert to review the patient imagery and generate the report for the physician:
information on the patient, information on the medical expert, information on previous reports generated from patient imagery, the medical expert's response to dialogue with the second virtual assistant, wherein the dialogue with the second virtual assistant comprises utilization of a VDU and/or a chatbot implemented by the first virtual assistant.

13. A medical imaging processing method (100), comprising:
supporting (110) by a first virtual assistant a physician to prepare an imaging request for a patient, wherein the imaging request is to be used by a technician who will carry out an imaging exam of the patient to generate patient imagery, and wherein the first virtual assistant utilizes one or more of the following to support the physician to prepare the imaging request:
information on the patient, information from a medical database, information on the physician,
information on previous imaging requests, the physician's response to dialogue with the first virtual assistant, wherein the dialogue with the first virtual assistant comprises utilization of a VDU and/or a chatbot implemented by the first virtual assistant;
providing the imaging request to the technician to carry out the imaging exam of the patient according to the imaging request to generate the patient imagery; and
supporting (130) by a second virtual assistant the medical expert to review the patient imagery and generate the report for the physician, and wherein the second virtual assistant utilizes one or more of the following to support the medical expert to review the patient imagery and generate the report for the physician:
information on the patient, information on the medical expert, information on previous reports generated from patient imagery, the medical expert's response to dialogue with the second virtual assistant, wherein the dialogue with the second virtual assistant comprises utilization of a VDU and/or a chatbot implemented by the second virtual assistant.

14. A medical imaging processing system (10), comprising:
- a first virtual assistant (30, 40); and
- a second virtual assistant (50);
wherein the first virtual assistant is configured to support a technician to carry out an imaging exam of a patient according to an imaging request prepared by a physician to generate patient imagery, and wherein the first virtual assistant is configured to utilize one or more of the following to support the technician to carry out the imaging exam of the patient according to the imaging request:
information on the patient, information on the technician, information on a medical expert who will review the patient imagery and generate a report for the physician; and
wherein the second virtual assistant is configured to support the medical expert to review the patient imagery and generate the report for the physician, and wherein the second virtual assistant is configured to utilize one or more of the following to support the medical expert to review the patient imagery and generate the report for the physician:
information on the patient, information on the medical expert, information on previous reports generated from patient imagery, the medical expert's response to dialogue with the third virtual assistant, wherein the dialogue with the third virtual assistant comprises utilization of a VDU and/or a chatbot implemented by the third virtual assistant.

15. A medical imaging processing method (100), comprising:
supporting (120) by a first virtual assistant a technician to carry out an imaging exam of a patient according to an imaging request prepared by a physician to generate patient imagery, and wherein the first virtual assistant utilizes one or more of the following to support the technician to carry out the imaging exam of the patient according to the imaging request:
information on the patient, information on the technician, information on a medical expert who will review the patient imagery and generate a report for the physician; and
supporting (130) by a second virtual assistant the medical expert to review the patient imagery and generate the report for the physician, and wherein the second virtual assistant utilizes one or more of the following to support the medical expert to review the patient imagery and generate the report for the physician:
information on the patient, information on the medical expert, information on previous reports generated from patient imagery, the medical expert's response to dialogue with the third virtual assistant, wherein the dialogue with the third virtual assistant comprises utilization of a VDU and/or a chatbot implemented by the third virtual assistant.
